# EUROPEAN PATENT SPECIFICATION

(11) **EP 3 681 509 B1**
(45) Date of publication and mention of the grant of the patent: **07.06.2023**
(21) Application number: 18711636.3
(22) Date of filing: 07.03.2018
(51) Int. Cl.: A61K 9/00, A61P 17/02, A61K 9/06, A61K 31/565, A61K 47/32, A61K 47/36, A61K 47/38, A61K 47/42, A61K 8/19, A61K 8/60, A61K 8/64, A61K 31/7004, A61K 33/244, A61K 47/34, A61K 8/73, A61K 33/38, A61K 45/06

(54) **WOUND HEALING MEDICAMENT**
WUNDHEILMITTEL
MÉDICAMENT CICATRISANT

(30) Priority: 07.03.2017 GB 201703656
(43) Date of publication of application: 22.07.2020
(73) Proprietor: The University Of Sheffield, Sheffield South, Yorkshire S10 2TN (GB); Comsats Institute Of Information Technology, Lahore (PK)
(72) Inventor: YAR, Muhammad, Lahore (PK); MACNEIL, Sheila, Sheffield S3 7HQ (GB)
(74) Representative: HGF
(86) International application number: PCT/GB2018/050579
(87) International publication number: WO 2018/162900

(56) References cited:
- EP-A1- 0 416 677
- WO-A1-2014/165253
- GB-A- 2 462 005
- MUHAMMAD YAR ET AL: "Deoxy-sugar releasing biodegradable hydrogels promote angiogenesis and stimulate wound healing", MATERIALS TODAY COMMUNICATIONS, vol. 13, 5 November 2017 (2017-11-05), pages 295-305, XP055475709, ISSN: 2352-4928, DOI: 10.1016/j.mtcomm.2017.10.015

## Description

This invention generally relates to the field of skin and hair restoration. More particularly, the present invention relates to the application of a chemical composition that promotes wound healing, angiogenesis, vascularisation and hair re-growth.

### BACKGROUND

There have been many studies looking at how to promote angiogenesis in wound beds. Of all the angiogenic factors, the most potent one commonly used in the development of biomaterials is vascular endothelial growth factor (VEGF) (Zhang et al., Biomaterials. 2015; Miyagi et al., Biomaterials. 2011; Khojasteh et al., Materials Science and Engineering: C. 2016; Johnson et al., Advances in Wound Care. 2014). This growth factor can be produced by recombinant technology and it has been examined critically in many studies (Neufeld et al., The FASEB journal. 1999; Long et al., Journal of theoretical biology. 2013; Xin et al., Cell. 2016). However, results have been generally disappointing when it is added on its own as it is rapidly broken down or diluted and washed away. More recent strategies have been to deliver VEGF bound to biomaterials. The biomaterials can take various forms including hydrogels, scaffolds or particles (Chiu et al., Biomaterials. 2010; Çak 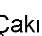 r-Özkan et al., Journal of Oral and Maxillofacial Surgery. 2017; Zhang et al., ACS Biomaterials Science & Engineering. 2016; Zhao et al., Advanced healthcare materials. 2016).

Several groups, including the MacNeil group, have looked to deliver VEGF from heparin bound to materials because heparin is a natural glycosaminoglycan present in the body at high concentrations in wounds where it acts to bind VEGF and other pro-angiogenic factors (Wu et al., Biomacromolecules. 2016; Gigliobianco et al., Journal of biomaterials applications. 2015). Thus the MacNeil group have developed materials where they have sought to immobilise heparin on an electrostatic basis either to hydrogels (Gilmore et al., Biotechnology and bioengineering. 2013) or in a layer by layer coating of electrospun scaffolds (Gigliobianco et al., Journal of biomaterials applications. 2015; Easton et al., Journal of Materials Chemistry B. 2014). *In vivo* heparin binds and releases VEGF and other pro-angiogenic mitogens (Ferrara N et al., Nature medicine. 2003). VEGF activates the proliferation and migration of endothelial cells in both normal and tumour tissue and results in rapid generation of new blood vessels (Harmey JH. Springer Science & Business Media; 2004; Hicklin et al., Journal of clinical oncology. 2005). Accordingly VEGF's potential has been explored in the preparation of materials where angiogenesis is required, for example in the preparation of synthetic dermis for the treatment of full thickness burns injuries (Tan et al., Journal of tissue engineering and regenerative medicine. 2014; Xie et al., Acta biomaterialia. 2013; Guo R et al., Biomaterials. 2011) and to stimulate wound healing in chronic non-healing wounds such as diabetic ulcers where the microvasculature is compromised.

Although VEGF stimulates angiogenesis it is very expensive (930$ for 50 µg from Sigma-Aldrich), and it is unstable (Simón-Yarza et al., Theranostics. 2012; Thompson et al., Oxford Textbook of Vascular Surgery: Oxford University Press; 2016). Other pro-angiogenic growth factors involved in new blood vessel formation are known and there is a complex cascade of factors that are produced and released in response to hypoxia.

The loss of barrier function of skin can be life-threatening (Chua et al., Burns & trauma. 2016; Blais et al., Stem cells translational medicine. 2013). The first-line treatment is usually the use of autologous split-skin grafts but in extensively burned patients there are insufficient grafts available to achieve rapid restoration of the barrier layer (Yi et al., Plastic and reconstructive surgery. 2015). Despite 30 years of research in this area there remains a need for a functional and cost-effective permanent dermal substitute to assist surgeons in managing patients with more than 30% full thickness wounding (Chua et al., Burns & trauma. 2016).

When burn wounds extend to more than 30 or 40% of the total body surface area then burns surgeons will seek to use natural and synthetic materials to provide immediate wound cover and aid in the eventual replacement of both the dermis and the epidermis (Sharma et al., Burns. 2014). It remains technically very difficult to produce a tissue engineered material that is equivalent to a split thickness skin graft (containing all of the epidermis and some of the dermis) that will "take" successfully on these wound beds (Böttcher-Haberzeth et al., Burns. 2010). The major challenge is not in the production of the materials in the laboratory (see Boyce *et al*) but in the tissue engineered materials surviving engraftment on the wound bed, which requires rapid ingrowth of new blood vessels from the underlying wound bed (Boyce et al., Annals of surgery. 2002; Supp et al., Clinics in dermatology. 2005). In practice, graft survival is entirely reliant on the growth of new blood vessels from the underlying wound bed into these tissue engineered materials which lack any intrinsic vasculature (Laschke et al., Tissue engineering. 2006; Hacker et al., Scientific reports. 2016).

In practice full thickness burns are usually treated in two stages (where insufficient autograft is available). Materials are used to provide a vascularised dermal substitute and then when this dermis is well vascularised then either a thin split thickness graft is placed on top of this (often by taking further skin grafts from the initial donor sites once healed) or by placing cultured cells over the vascularised dermis. The two most commonly used materials to provide a vascularised dermis are a biomaterial, Integra, which has been developed for this purpose and donor cadaveric skin (Nguyen et al., Burns. 2010; Weigert et al., Journal of Hand Surgery (European Volume). 2011; Cleland et al., Burns. 2014). Integra is composed of a substrate of bovine collagen with shark chondroitin sulphate onto which a silicon membrane has been bonded (Chua et al., Burns & trauma. 2016). The burnt tissue is excised clinically, Integra put in place and then left in place until it becomes vascularised which often takes three weeks or more. At this point surgeons can remove the silicon barrier membrane and place on top of it a thin split thickness skin graft. The alternative material that is used is cadaveric skin. This can be used to provide immediate wound cover (once the burnt tissue is excised) and restore the barrier function. It then becomes vascularised within the space of a few weeks and the donor epidermis can then be gently removed leaving the donor vascularised dermis in place and the epidermal barrier is replaced with a split thickness skin graft from the patient or with epidermal cells cultured from the patient (as discussed in MacNeil, Nature. 2007). However, these materials are not always available to burns surgeons throughout the world because of the lack of well-run skin banks (for donor skin) or because the purchase of Integra is viewed as too expensive.

Hair loss is a common condition in humans and is characterised by the loss or reduction in the amount of hair from the head or body. A number of different conditions can involve hair loss. Whilst there are known causes of some types of hair loss including infection, drugs, trauma and pregnancy the cause of other types of hair loss remains unknown. Conventional therapies for some hair loss may include drug therapy, for example with medications such as minoxidil or finasteride. Another therapy used is hair transplant surgery, which involves harvesting hair follicles with hair fibres from a portion of the body with hair and re-locating the hair follicles to a portion of the body that is devoid of hair. Patent document WO 2014/165253 generally discloses wound healing to be promoted by carbohydrates, monosaccharaides, disaccharides and polysaccharides.

There remains a need for new effective and affordable biomaterials to provide a well vascularised dermal substrate in the management of major burns. The key issue is that none of the current materials contain any intrinsic vasculature hence ingrowth of vessels depends entirely on the blood vessels in the underlying wound bed (Supp et al., Clinics in dermatology. 2005; Sahota et al., Wound repair and regeneration. 2003). There remains the need for a dermal substrate that allows for improved vascularisation leading to improved post implantation survival.

### BRIEF SUMMARY OF THE DISCLOSURE

In one aspect the invention provides a D-deoxyribose sugar for use in promoting wound healing wherein the sugar is provided in a carrier and wherein the carrier is a biocompatible matrix material or a hydrogel. Preferably, the D-deoxyribose is 2-deoxyribose.

In one embodiment, the carrier is a biodegradable carrier.

In one embodiment, the matrix material is an electrospun scaffold. Preferably, the electrospun scaffold comprises at least one of polylactic acid (PLA), polyglycolide (PGA), poly(lactic-co-glycolic acid) (PLGA) or poly(3-hydroxybutyrate-co-3-hydroxyvalerate) PHBV.

In one embodiment, the hydrogel is a crosslinked hydrogel. Preferably the hydrogel comprises at least one of chitosan, gelatin, alginate, agarose, methylcellulose, hyaluronan or any combination thereof. In one embodiment, the hydrogel comprises chitosan and collagen. Additionally, the hydrogel may comprise polyvinyl alcohol, sodium polyacrylate, acrylate polymers or any combination thereof. In one embodiment, the hydrogel comprises chitosan and polyvinyl alcohol.

In one embodiment, the carrier further comprises an antimicrobial agent.

In one embodiment, the wound is a chronic wound.

In one embodiment, the wound is a full thickness wound.

In one embodiment, the wound is a burn injury.

In one aspect, the invention provides a biocompatible matrix material comprising a D-deoxyribose sugar.

In one embodiment, the matrix material is an electrospun scaffold. Preferably, the electrospun scaffold comprises at least one of polylactic acid (PLA), polyglycolide (PGA), poly(lactic-co-glycolic acid) (PLGA) or poly(3-hydroxybutyrate-co-3-hydroxyvalerate) PHBV.

In one aspect the invention provides a hydrogel comprising a D-deoxyribose sugar.

In one embodiment, the hydrogel is a crosslinked hydrogel. Preferably the hydrogel comprises at least one of chitosan, gelatin, alginate, agarose, methylcellulose, hyaluronan or any combination thereof. In one embodiment, the hydrogel comprises chitosan and collagen. Additionally, the hydrogel may comprise polyvinyl alcohol, sodium polyacrylate, acrylate polymers or any combination thereof. In one embodiment, the hydrogel comprises chitosan and polyvinyl alcohol.

In one aspect, the invention provides the biocompatible material of any one the aforementioned aspects or embodiments for use in promoting wound healing or for use in the treatment of alopecia.

In one aspect, the invention provides the hydrogel of any one of the aforementioned aspects or embodiments for use in promoting wound healing or for use in the treatment of alopecia.

In one aspect, the invention provides the biocompatible material of any one the aforementioned aspects or embodiments for use in a method of increasing or inducing vascularization in a wound bed.

In one aspect, the invention provides the hydrogel of any one of any one the aforementioned aspects or embodiments for use in a method of increasing or inducing angiogenesis in a wound bed.

In one aspect the invention provides a D-deoxyribose sugar for use in the treatment of alopecia wherein the sugar is provided in a carrier and wherein the carrier is a biocompatible matrix material or a hydrogel. Preferably, the D-deoxyribose is 2-deoxyribose.

In one embodiment, the carrier is a biodegradable carrier.

In one embodiment, the matrix material is an electrospun scaffold. Preferably, the electrospun scaffold comprises at least one of polylactic acid (PLA), polyglycolide (PGA), poly(lactic-co-glycolic acid) (PLGA) or poly(3-hydroxybutyrate-co-3-hydroxyvalerate) PHBV.

In one embodiment, the hydrogel is a crosslinked hydrogel. Preferably the hydrogel comprises at least one of chitosan, gelatin, alginate, agarose, methylcellulose, hyaluronan or any combination thereof. In one embodiment, the hydrogel comprises chitosan and collagen. Additionally, the hydrogel may comprise polyvinyl alcohol, sodium polyacrylate, acrylate polymers or any combination thereof. In one embodiment, the hydrogel comprises chitosan and polyvinyl alcohol.

In one embodiment, the carrier further comprises an antimicrobial agent.

In one aspect, the invention provides a non-therapeutic method for promoting hair regrowth, said method comprising administration of a D-deoxyribose sugar, wherein the sugar is provided in a carrier and wherein the carrier is a biocompatible matrix material or a hydrogel Preferably, the D-deoxyribose is 2-deoxyribose.

In one embodiment, the carrier is a biodegradable carrier.

In one embodiment, the matrix material is an electrospun scaffold. Preferably, the electrospun scaffold comprises at least one of polylactic acid (PLA), polyglycolide (PGA), poly(lactic-co-glycolic acid) (PLGA) or poly(3-hydroxybutyrate-co-3-hydroxyvalerate) PHBV.

In one embodiment, the hydrogel is a crosslinked hydrogel. Preferably the hydrogel comprises at least one of chitosan, gelatin, alginate, agarose, methylcellulose, hyaluronan or any combination thereof. In one embodiment, the hydrogel comprises chitosan and collagen. Additionally, the hydrogel may comprise polyvinyl alcohol, sodium polyacrylate, acrylate polymers or any combination thereof. In one embodiment, the hydrogel comprises chitosan and polyvinyl alcohol.

In one embodiment, the carrier further comprises an antimicrobial agent.

In one aspect, the invention provides a wound dressing comprising the biocompatible matrix material according to any one of the aforementioned aspects or embodiments.

In one aspect, the invention provides a wound dressing comprising the hydrogel according to any one of the aforementioned aspects or embodiments.

### BRIEF DESCRIPTION OF THE DRAWINGS

Embodiments of the invention are further described hereinafter with reference to the accompanying drawings, in which:

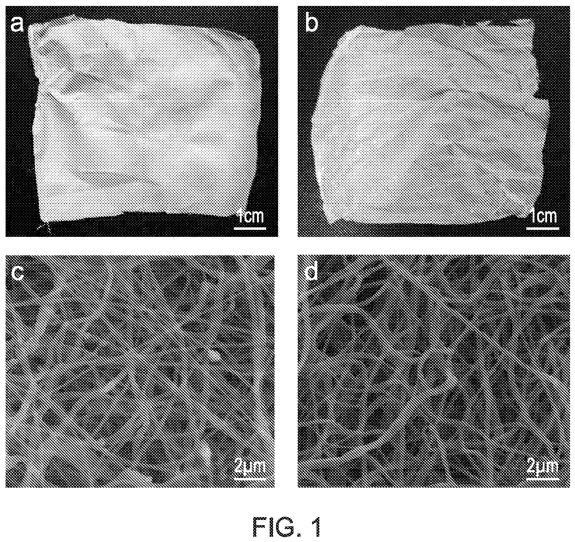

Figure 1 shows an optical and Scanning Electron Microscope (SEM) images of PCL electrospun fibers. Optical images of fibrous sheets (a) without and (b) with D-deoxyrobose. SEM micrographs of fibers without (c) and with (d) D-deoxyribose at 10000X magnification.

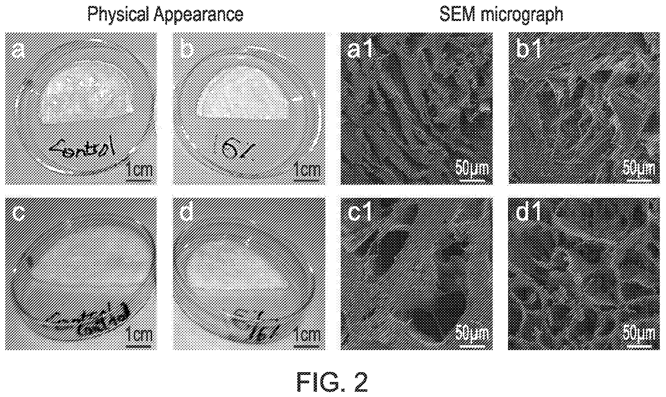

Figure 2 shows the physical appearance of hydrogels (left) and their SEM micrographs (right). (a) freeze dried hydrogels without TEOF (b) freeze dried hydrogels with 16% TEOF; (c) physical loading of sugar on hydrogel without TEOF (d) physical loading of sugar on hydrogel with 16% TEOF; (a1) SEM micrograph of hydrogels without TEOF magnification 500X (b1) SEM micrograph of hydrogels with 16% TEOF, magnification 500X (c1) SEM micrograph of sugar-loaded hydrogel without TEOF, magnification 500X (d1) SEM micrograph of sugar-loaded hydrogel with 16% TEOF, magnification 500X.

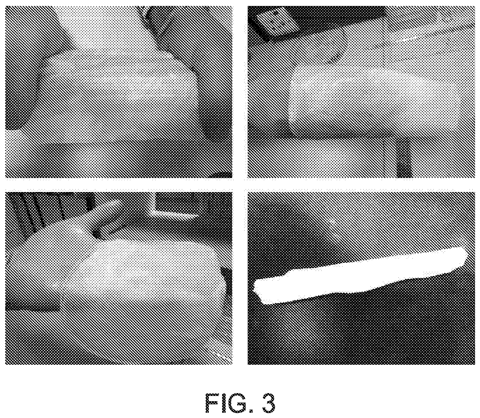

Figure 3 shows photographs of chitosan/collagen hydrogels to show their strength, shape, flexibility, and ability to be folded.

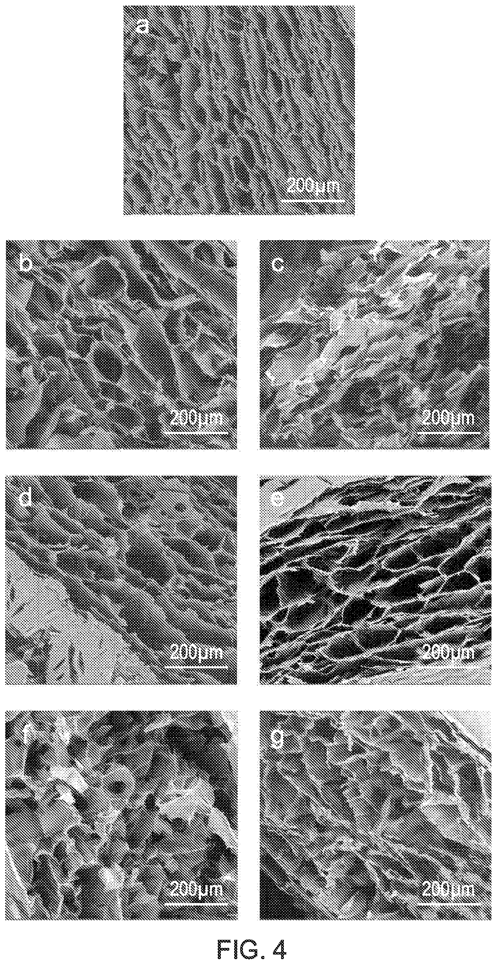

Figure 4 shows scanning Electron Microscope (SEM) micrographs of crosslinked CS/Collagen scaffold showing cross-sectional view of hydrogels. Micrographs revealing porous structure of the hydrogels, Magnification 200X; (a) control (without sugars), (b) D-deoxyribose loaded hydrogel, (c) L-deoxyribose loaded hydrogel, (d) D-deoxyrhamnose loaded hydrogel, (e) L-deoxyrhamnose loaded hydrogel, (f) D-deoxyfucose loaded hydrogel, (g) D-deoxyfucose loaded hydrogel.

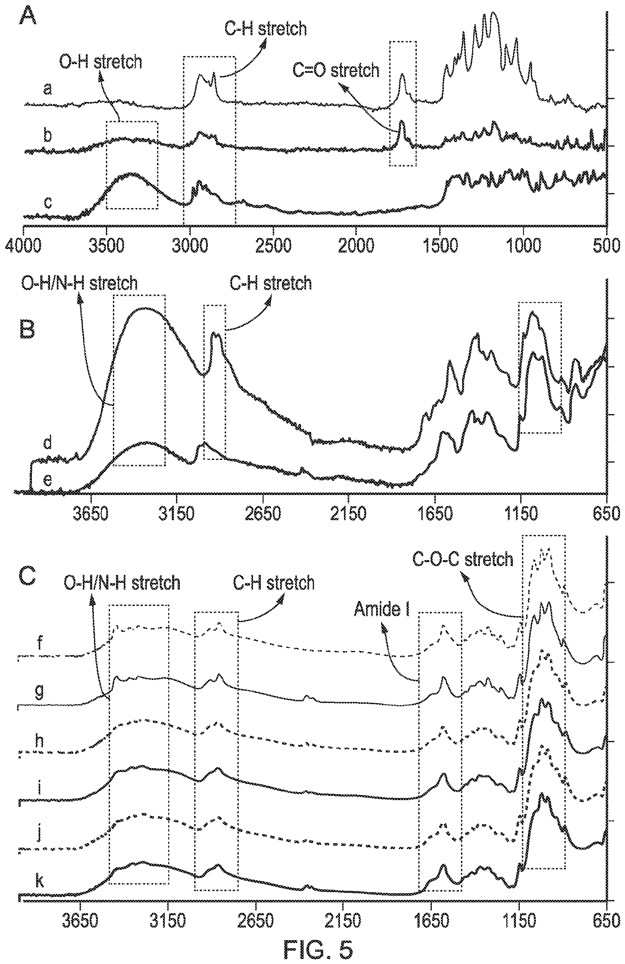

Figure 5 shows FTIR spectra of different synthesized materials [A] Electrospun fibers (a) PCL fibres (b) D-ribose-loaded electrospun PCL fibers (c) D-deoxyribose; [B] FTIR spectra of CS/PVA hydrogels (d) control without TEOF TEOF (e) D-deoxyribose loaded hydrogel with 16% TEOF; [C] FTIR of CS/Collagen/TEOF hydrogels (f) D-deoxyfucose loaded hydrogel, (g) L-deoxyrhamnose loaded hydrogel, (h) D-deoxyrhamnose loaded hydrogel, (i) L-deoxyribose loaded hydrogel, (j) D-deoxyribose loaded hydrogel, (k) control (without sugars).

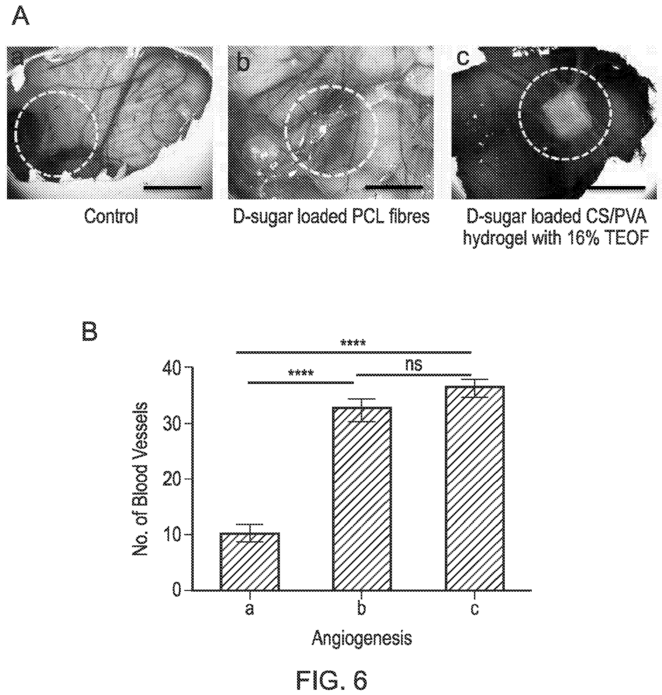

Figure 6 shows [A] Assessment of angiogenic potential of D-ribose loaded electrospun fibers and hydrogels (a) PCL e-spun fibers without D-deoxyribose (b) PCL e-spun fibers with D-deoxyribose (c) D-deoxyribose loaded CS/PVA hydrogel with 16% TEOF; [B] Statistical histogram showing number of blood vessels penetrating the scaffolds. A circle was drawn 1mm away from the scaffold and number of blood vessels was counted inside the circle. For each study, 8 eggs were implanted with scaffolds and on average of 5 chicks survived. The results are mean ± S.D of 5 separate eggs.

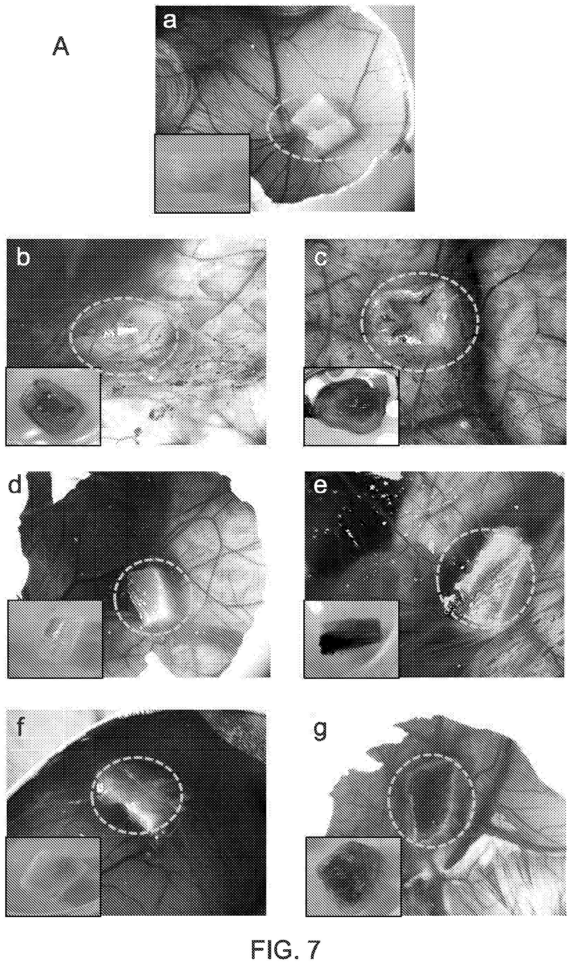

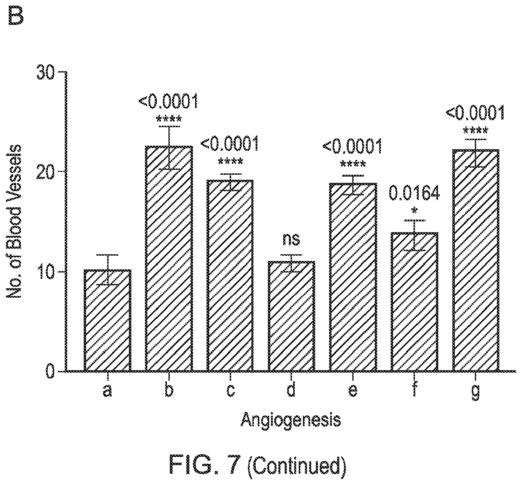

Figure 7 shows the proangiogenic potential of CS/Collagen/TEOF hydrogels using the CAM assay. [A] The scaffolds were placed onto the CAM at day 8 of fertilization. The figure shows light microscope images of scaffolds on CAM at day 14 of fertilization (a) control (without sugars), (b) D-deoxyribose loaded hydrogel, (c) L-deoxyribose loaded hydrogel, (d) D-deoxyrhamnose loaded hydrogel, (e) L-deoxyrhamnose loaded hydrogel, (f) D-deoxyfucose loaded hydrogel, (g) L-deoxyfucose loaded hydrogel. The figure also shows respective explanted hydrogels in the lower left corner of each picture. For each experiment 10 eggs were implanted with the respective scaffolds out of which 7 chicks survived. [B] The lower part of the picture shows statistical analysis of the number of blood vessel for each hydrogel. (a) control (without sugars), (b) D-deoxyribose loaded hydrogel, (c) L-deoxyribose loaded hydrogel, (d) D-deoxyrhamnose loaded hydrogel, (e) L-deoxyrhamnose loaded hydrogel, (f) D-deoxyfucose loaded hydrogel, (g) D-deoxyfucose loaded hydrogel. Statistical p values are given above the graphical bars. The values shown are means ± S.D of seven chicks for each scaffold.

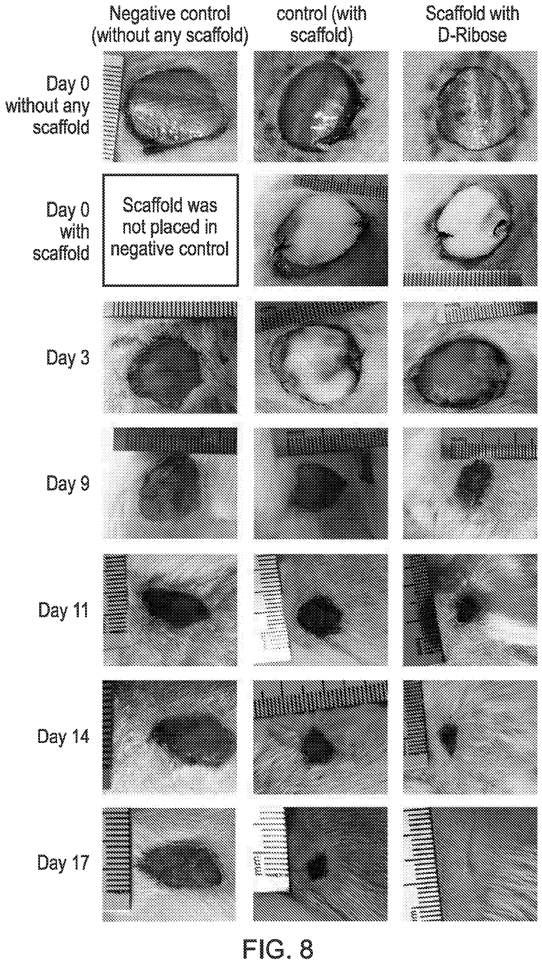

Figure 8 shows wound healing was accelerated by D-deoxyribose loaded scaffolds. Full-thickness wounds were created by marking a 20mm area and then excisions were performed under anaesthetic using surgical scissors. The respective materials were applied to wounds, sutured in place and initially covered with Mepore (Sweden) adhesive bandages and finally with cotton bandages. All rats received the same dressings which were removed 3 days post-wounding. Each wound was photographed digitally at specific time points and further quantified using Image J software. Representative macroscopic pictures of wounds, control hydrogels (cross-linked CS/collagen scaffold) and D-ribose loaded hydrogels are shown at days 3, 9, 11, 14 and 17 post-wounding.

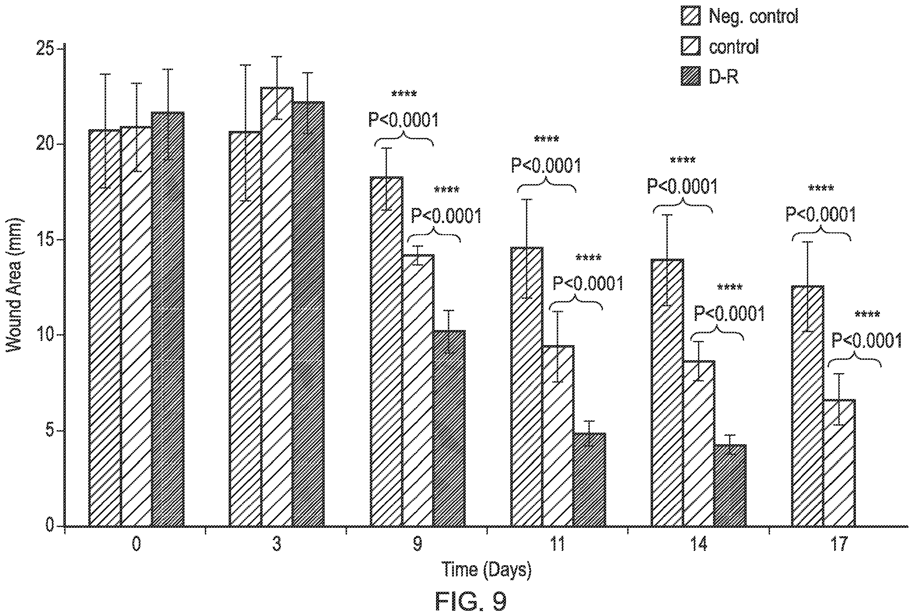

Figure 9 shows wound areas over 17 days are shown in the histograms. Full-thickness wounds were created by marking out 20mm circular areas and then excising them with surgical scissors. Negative control was used which was actually open wound and was left to heal naturally. Control contained CS/Collagen scaffold without any sugar loading and D-R was CS/Collagen scaffold loaded with D-deoxyribose. For each type of experiment three rats were used and wound healing was observed till complete wound closure. The average diameter of the wound was determined using Image J and each column represents the mean ± S.D of independent calculations recorded from four wounds for each sample. Statistical analysis showed that unloaded hydrogels significantly accelerated wound healing and the addition of D-deoxyribose greatly accelerated wound healing as can be seen by days 9, 11, 14 and 17. p<0.0001.

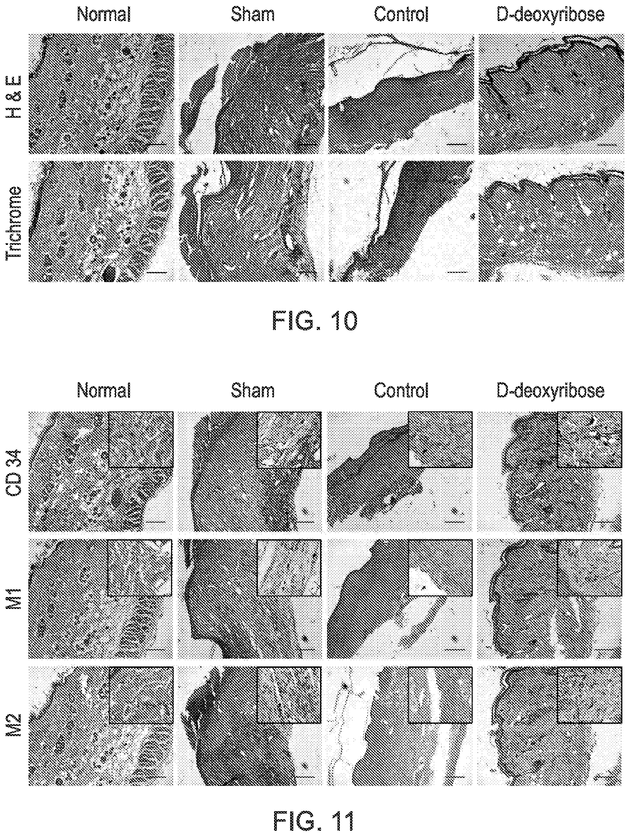

Figure 10 shows H&E and Goldren-trichrome images at day 17. Scale bars of 0.2 mm. Figure 11 shows immunostaining images at day 17. Scale bars of 0.2 mm.

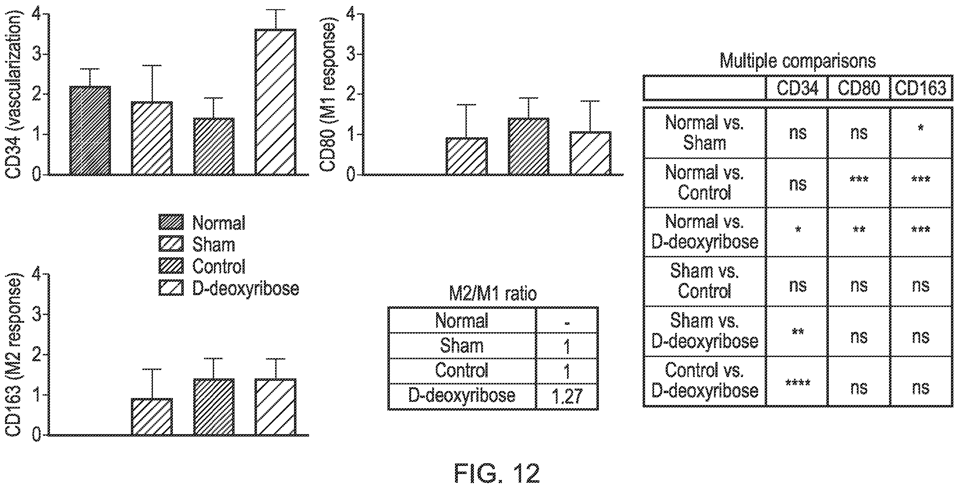

Figure 12 shows an assessment of the immunostaining data using a blind scoring system. 0 = absence; 1 = mild presence; 2 = large presence; 3 = abundance; 4 = great abundance. Results shown are means ± SD, n=10. Statistics for multiple comparisons and the M2/M1 ratio are shown too.

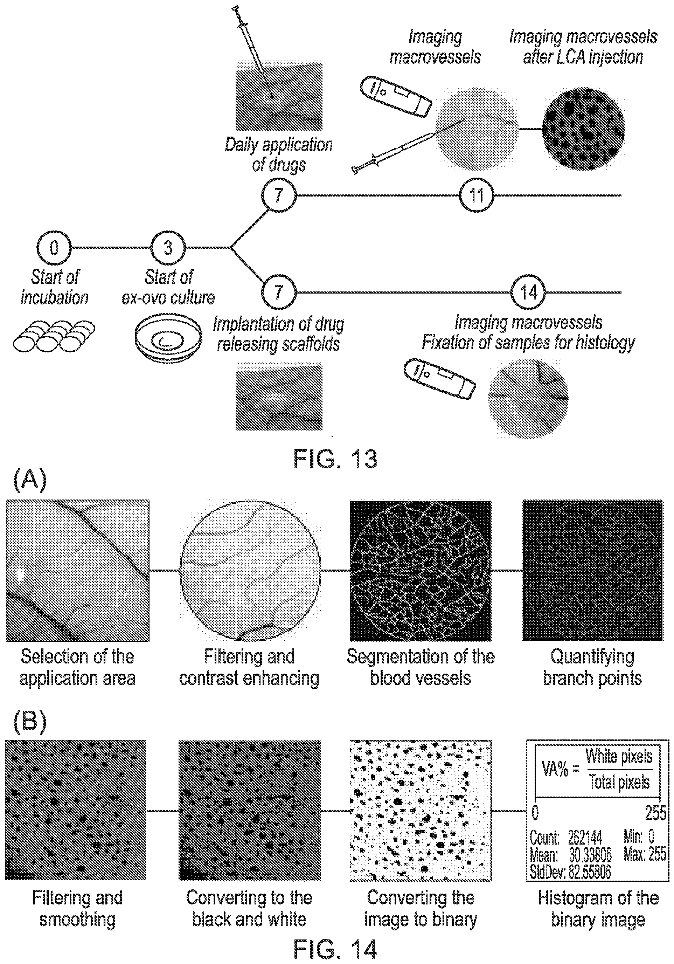

Figure 13 shows a schematic illustration of the steps of the direct application of the substances and implantation of the substance releasing scaffolds on CAM. Figure shows the basic methodology of *ex-ovo* CAM assay starting from EDD0.
Figure 14 shows steps demonstrating the quantification method of macrovasculature (A) and microvasculature (B) of CAM.

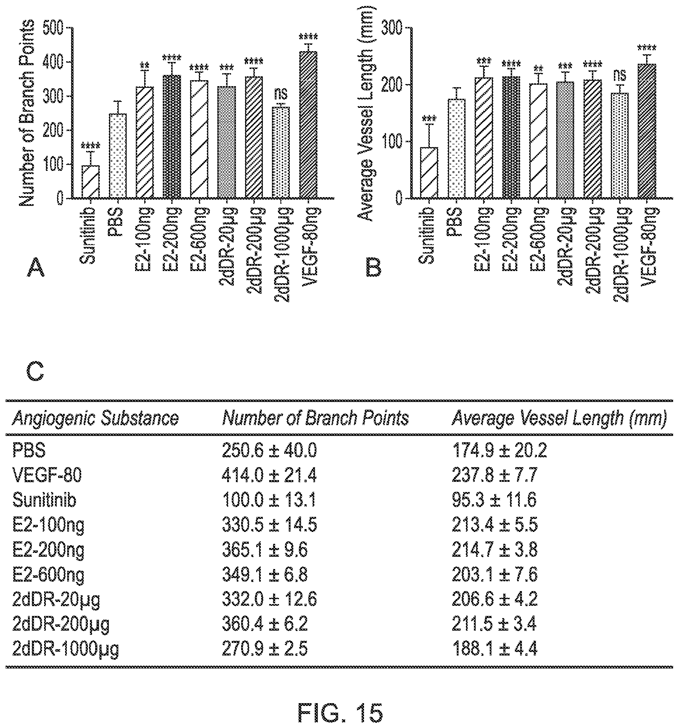

Figure 15 shows a comparison of the angiogenic response to E2 and 2dDR assessed using the CAM assay. (A) number of branch points, (B) average vessel length. **** P ≤ 0.0001, *** P ≤ 0.001, ** P ≤ 0.01, * P ≤ 0.05, ^{ns} P≥0.05, n=9±SD. The number of branch points and average macrovessel lengths seen in response to different concentrations of E2 and 2dDR were calculated and compared to PBS controls over 4 days (C). Values represent mean ± SD.

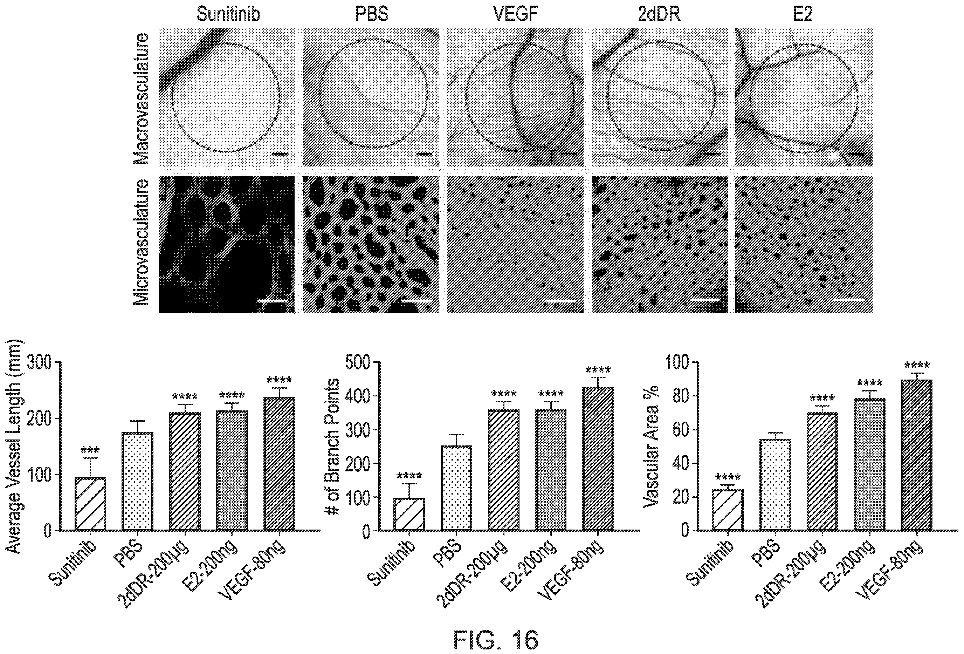

Figure 16 shows a demonstration of the effect of E2 (200ng/day) and 2dDR(200µg/day) on macrovasculature and microvasculature compared to the application of PBS (Control). VEGF was included as a positive control and Sunitinib as a negative control. 2dDR was applied as 200µg/day and E2 as 200 ng/day. All statistical comparisons are made to PBS. **** P ≤ 0.0001, *** P ≤ 0.001. Scale bars represent 1 mm and 50 µm respectively for images of macrovessels (on the top) and microvessels (in the middle), n=9±SD.

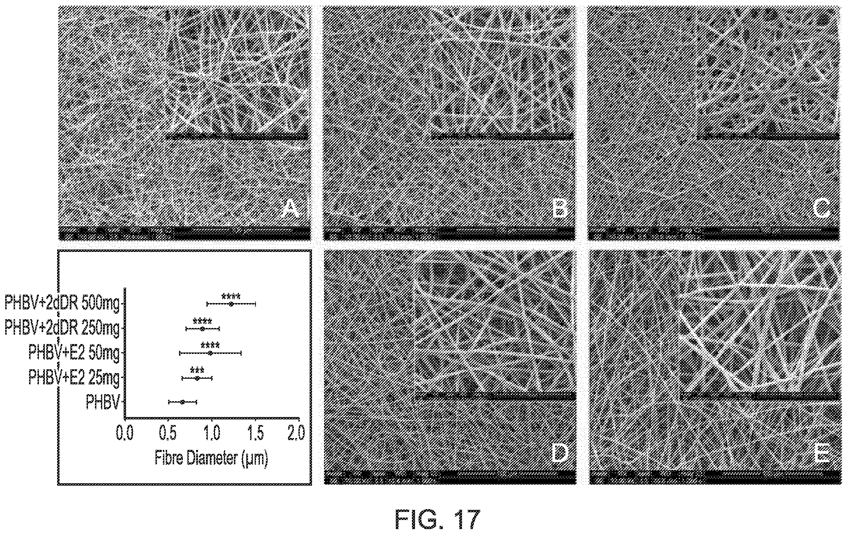

Figure 17 shows SEM images of the scaffolds. (A) Plain PHBV, (B) PHBV + 25 mg E2, (C) PHBV + 50 mg E2, (D) PHBV + 250 mg 2dDR, (E) PHBV + 500 mg 2dDR. The graph on the bottom left corner shows the distribution of fibre diameters for each scaffold, **** P ≤ 0.0001, *** P ≤ 0.001. Scale bars represent 100 µm.

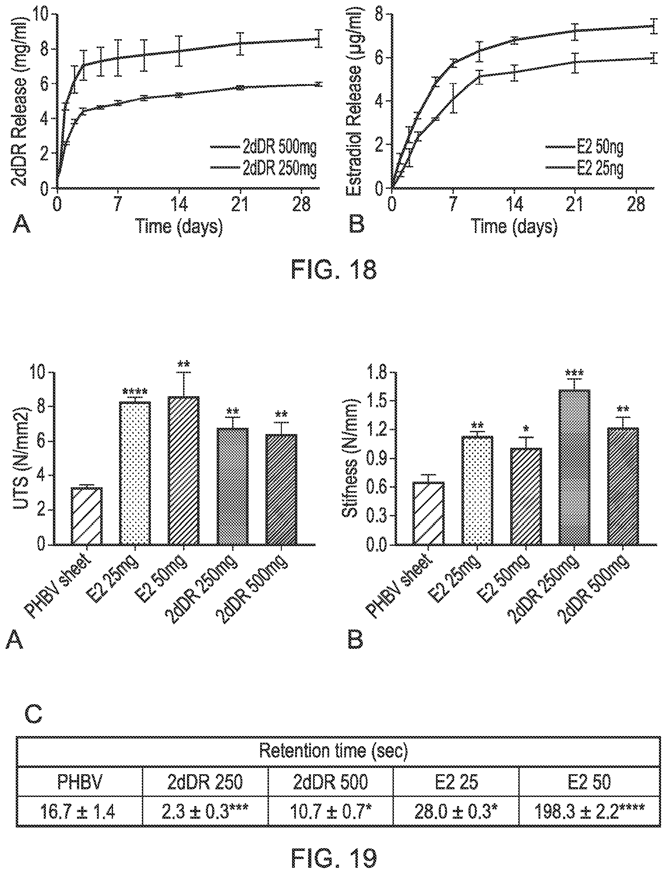

Figure 18 shows release of (A) 2dDR and (B) E2 from PHBV scaffolds over 30 days, n=6±SD. Figure 19 shows comparison of (A) UTS, (B) stiffness and (C) droplet retention time on the scaffolds, **** P ≤ 0.0001, *** P ≤ 0.001, ** P ≤ 0.01, * P ≤ 0.05, n=6±SD.

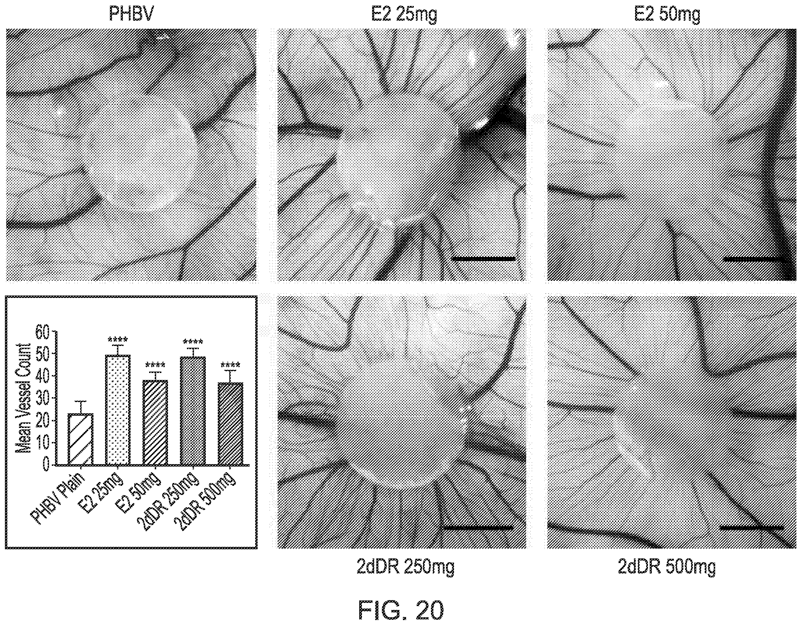

Figure 20 shows representative images demonstrating the angiogenic potential of 2dDR (250 mg and 500 mg) and E2 (25 mg and 50 mg) releasing scaffolds in comparison with PHBV scaffolds. The graph on the bottom left shows the quantitative data from these experimentsmean vessel counts, **** P ≤ 0.0001. Scale bars represent 3mm, n=6±SD.

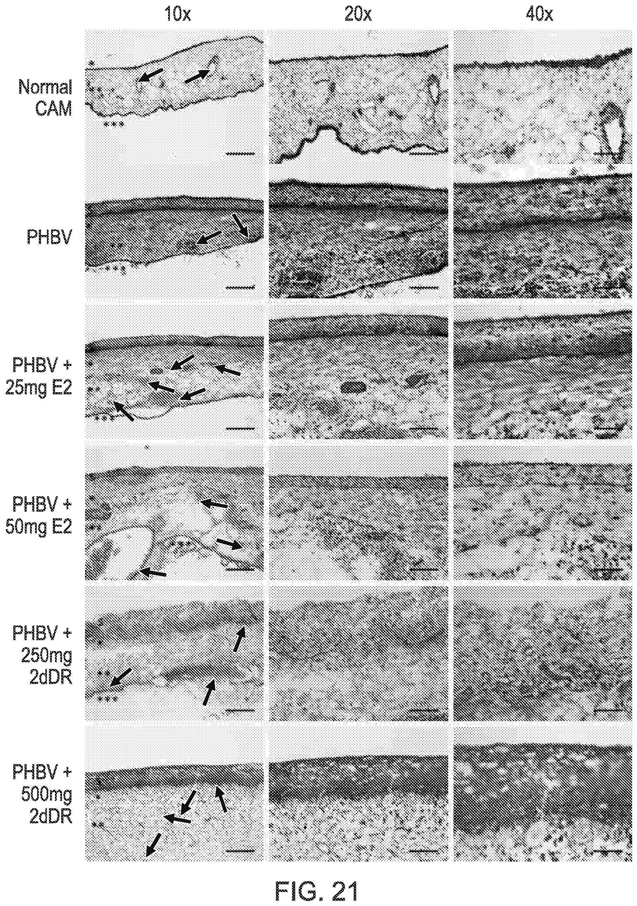

Figure 21 shows histological analysis of CAMs after 7 days of incubation with or without scaffolds in different magnifications. Orientation of the scaffold (s), CAM Ectoderm (*), mesoderm (**), and endoderm (***) layers were indicated in the images. Arrows shows the blood vessels. Scale bars = 0.2 mm 10×), 0.1 mm (20×), 0.05 mm (40×).

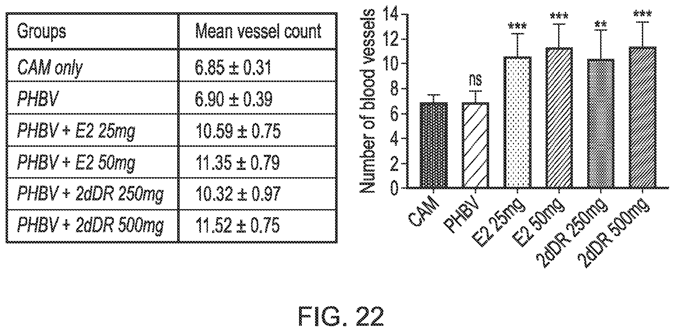

Figure 22 shows quantification of the discernible blood vessels adjacent to the scaffolds at 10× magnification from the total of six different slides for each group and six different area of interest from each slide.

### DETAILED DESCRIPTION

The inventors have surprisingly found that biomaterials loaded with d-deoxyribose or L-deoxy sugars (not claimed) supported the rapid formation of new blood vessels and aided wound healing. A hydrogel loaded with deoxyribose also stimulated healing in an *in vivo* wound model. Wound healing was associated with new blood vessel formation. The ability of d-deoxyribose to promote wound healing is particularly surprising given that other D-deoxy sugars, such as 2-deoxy-D-glucose are known to inhibit angiogenesis (Merchan J. et al., PLoS ONE 5(10): e13699).

The data presented shows that biomaterials loaded with 2-deoxy-D-ribose supported the formation of new blood vessels within 7 days. This angiogenic property of deoxyribose was not shared to any useful degree by two other deoxy sugars investigated, namely -deoxy fucose and deoxy rhamnose but whereas L isomers of all three sugars were strongly angiogenic only the D isomer of deoxyribose was strongly angiogenic. There are several advantages to using 2-deoxy-D-ribose or an L-deoxy sugar to stimulate VEGF production. For example, they are stable and inexpensive and can be introduced into biomaterials to give sustained release over several days to stimulate the growth of new blood vessels. In addition most bacteria can find it difficult to metabolise deoxyribose (Christensen et al., Journal of bacteriology. 2003).

The inventors have also found that common Gram-positive pathogens were unable to metabolise any of the D or L isomers of the three deoxy-sugars tested (-deoxy ribose, -deoxy fucose and deoxy rhamnose) but some of these sugars could be metabolised by a Gramnegative pathogenic species.

The inventors have demonstrated that a hydrogel of chitosan/collagen hydrogel loaded with 2-deoxy-D-ribose stimulated healing in an *in vivo* rat cutaneous wound model. Wound healing was associated with new blood vessel formation.

The inventors demonstrated that the gradual release of 2-deoxy-D-ribose from biomaterials will promote the production of new blood vessels and hence be of value in wound healing.. The inventors introduced a D sugar, 2-deoxy-D-ribose to see if it could prove pro-angiogenic and to assess whether this could be done using a range of biomaterials of clinical relevance. Accordingly this D-deoxy sugar was loaded into three biodegradable biomaterials, electrospun PCL nanofibers, hydrogels of chitosan/PVA, and hydrogels of chitosan/collagen.

The data show that this D sugar, 2-deoxy-D-ribose, can be readily incorporated into three different biomaterials and the inventors have confirmed that it was indeed proangiogenic using the CAM assay. Also using this assay the inventors explored to what extent the angiogenic properties of the D isomer of deoxy ribose were shared with other deoxy sugars. Here the inventors found that all three L isomers of deoxy sugars (ribose, fucose and rhamnose) were proangiogenic but only the D isomer of deoxyribose was significantly angiogenic.

Looking at the ability of these sugars, to act as metabolic substrates for bacteria, the inventors looked at three strains of *Staphlococcus aureus* but none of them were able to metabolise these sugars. This is encouraging news in seeking to develop biomaterials containing these sugars as they are not a source of nutrients for bacteria.

The contribution of the D isomer of deoxy-ribose to wound healing was assessed *in vivo,* using a chitosan/collagen hydrogel. For this the inventors used a rat cutaneous wound model. The inventors surprisingly found that the addition of D-deoxy-ribose to a chitosan/collagen hydrogel greatly accelerated cutaneous wound healing associated with an increase in vascularisation as detected by staining for CD34 positive cells. It was clear from this model that the chitosan collagen gel itself stimulated wound healing but this was greatly increased by the addition of the D sugar. By day 17 the D sugar treated wounds had completely closed and histology showed the presence of very mature hair follicles. On the basis of this evidence the inventors conclude that the release of D sugar from a chitosan /collagen gel stimulates angiogenesis and we propose that the increased wound healing is a natural consequence of this.

Examination of the macrophage response to the deoxy sugar showed a slight preponderance of M2 macrophages over M1 macrophages by day 17 indicative of constructive remodelling. In conducting the animal experiments the initial hydrogel was quite robust to handle and indeed could be sutured in place. Animal trial observations at day 3 revealed that the hydrogels were very well-attached and ingrowth of cells was also evident **(****Figure** 8). The attachment of hydrogels with surrounding tissue was better in the case of the sugar-loaded hydrogel. At day 9, control and D-deoxyribose loaded hydrogels were still intact. The hydrogels were gradually absorbed and by day 11 more than 50% of the wound area was healed with the D-deoxyribose hydrogel (see **Figure** 9) but the remaining hydrogel was still present on the wound **(****Figure** 8). By day 14 there was no sign of D-deoxysugar hydrogel remaining on the wound. By day 17, the D-deoxyribose grafted wound had healed completely. This hydrogel appeared to be totally absorbed by the animal and could not be felt inside the healed skin when palpating. The control hydrogel maintained its integrity until the end of the animal study.

These deoxy sugar-releasing hydrogels are a promising approach to stimulate angiogenesis in chronic non-healing wounds and also to act as a dermal replacement material in the management of extensive full thickness burns.

There are several advantages to using this D-sugar to stimulate VEGF production; it is stable and inexpensive and can be introduced into biomaterials to give sustained release over several days to stimulate the growth of new blood vessels.

The inventors have found that a chitosan-based hydrogel releasing D-deoxy ribose could act as a substitute for a dermis as it will stimulate new blood vessel ingrowth and provide a vascularised wound bed for subsequent grafting with a thin skin graft or cultured autologous keratinocytes to provide a permanent skin barrier layer.

The positive effect of D-deoxy ribose is surprising as in 2010, Merchan *et al.* reported antiangiogenic activity of 2-deoxy-D-Glucose (2-DG). They reported that, 2-DG inhibited endothelial capillary formation and endothelial cell migration *in vitro* [.R. Merchan, K. Kovács, J.W. Railsback, M. Kurtoglu, Y. Jing, Y. Piña, N. Gao, T.G. Murray, M.A. Lehrman, T.J. Lampidis, Antiangiogenic activity of 2-deoxy-D-glucose, PLoS One. 5 (2010). doi:10.1371/journal.pone.0013699].

The data presented herewith demonstrates that for a clinically useful product rapid (within 5 days) neovascularisation post implantation is critical. Rapid ingrowth and infiltration of blood vessels is crucial for any TE constructs greater than 200 µm to be able to survive *in vivo* [C.K. Griffith, C. Miller, R.C.A. Sainson, J.W. Calvert, N.L. Jeon, C.C.W. Hughes, S.C.George, Diffusion Limits of an in Vitro Thick Prevascularized Tissue, Tissue Eng. 11 (2005) 257-266. doi:10.1089/ten.2005.11.257]. The inventors have overcome this barrier by functionalise a tissue engineered carrier with proangiogenic substances.

The inventors have produced tissue engineered carriers that overcome the delayed neovascularisation commonly seen post transplantation of tissue engineered constructs.

The inventors have demonstrated that 2dDR loaded CS/collagen hydrogels were firmly attached to the wound bed of full thickness excisional wounds (20mm) in rats by day 3 and wounds treated with 2dDR, were completely closed by day 17 with clear hair growth while control wounds remained open with no evidence of epithelial tissue or hair growth. The histology of the wound beds of all animals sacrificed at day 17, showed complete healing in the case of 2dDR treated wounds with well-developed hair follicles and the presence of new blood vessels in the healed wounds was confirmed with CD34 staining [M. Yar, L. Shahzadi, M. Azra, M.I. Raheem, S. Roman, A.A. Chaudhry, I. ur Rehman, C.W.I. Douglas, S. MacNeil, Deoxy-sugar releasing biodegradable membranes and hydrogels promote angiogenesis and stimulate wound healing, Mater Today Commun. 13 (2017) 295-305. doi:10.1016/j.mtcomm.2017.10.015].

Carbohydrates can have D- and L- enantiomeric configurations which are determined by the configuration of the chiral carbon that is furthest from the anomeric carbon. When the linearized form of a carbohydrate is drawn as a Fischer projection, the hydroxyl group bonded to the chiral carbon may be positioned to either the left or right of the carbon. If the hydroxyl is to the right of the chiral carbon then the carbohydrate is annotated as a D-carbohydrate whereas if the hydroxyl group is to the left of the chiral carbon the carbohydrate is annotated as an L-carbohydrate. The following example illustrates the configurations of the D- and L-enantiomeric forms of glucose:

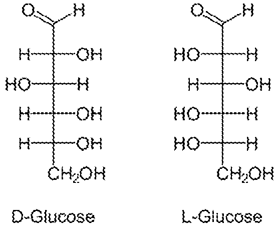

The D-enantiomers of carbohydrates are the most prevalent form found to occur in nature and many can be isolated from natural sources. In contrast, non-naturally occurring L-enantiomers of carbohydrates can be expensive to manufacture by chemical or enzymatic synthesis. However, L-enantiomers of some carbohydrates are also known to occur in nature.

One particular example of a naturally occurring D-sugar is D-ribose (chemical formula C₅H₁₀O₅): 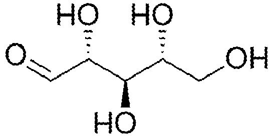 linearised form of D-ribose Phosphorylated forms of D-ribose are involved in amino acid synthesis, used in the pentose phosphate pathway and are constituents of ribonucleic acid (RNA).

Suitably, D-ribose can also be de-hydroxylated, for example at the 2', 3', 4' or 5' position. One notable example of a de-hydroxylated ribose is 2-D-deoxyribose wherein the hydroxyl group at the 2' position replaced with a Hydrogen atom. This sugar is a constituent of deoxyribonucleic acid (DNA). Deoxy-D-ribose exists predominantly as a cyclic form in solution rather than in a linearised form: 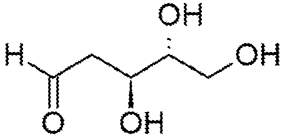 Linearised form of 2-D-deoxyribose 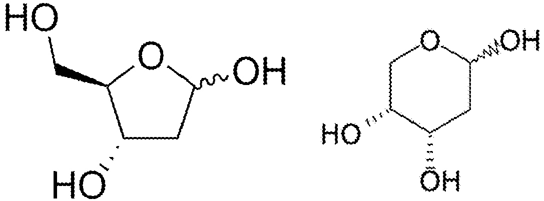 Cyclic forms of 2-D-deoxyribose D-enantiomers of deoxy ribose find particular use in the invention.

Deoxy sugars are sugars that have a hydroxyl group replaced with a hydrogen atom. Examples of deoxy sugars include L-2 deoxyribose, L-fucose (equivalent to 6-deoxy-L-galactose) and L-rhamnose (equivalent to 6-deoxy-L-mannose),

L-enantiomers of deoxy sugars find particular use in the disclosure, but are not claimed.

Also disclosed, but not claimed, is that the inventors have also surprisingly found that biomaterials loaded with Oestradiol (E2) supported the rapid formation of new blood vessels and aided wound healing. Oestradiol (E2) has an important role in neovascularisation during the menstrual cycle [D.W. Losordo, J.M. Isner, Estrogen and Angiogenesis: A Review, Arterioscler 30 Thromb Vasc Biol. 21 (2001) 6-12. doi:10.1161/01.ATV.21.1.6, Y. Matsubara, K. Matsubara, Estrogen and progesterone play pivotal roles in endothelial progenitor cell proliferation, Reprod Biol Endocrinol. 10 (2012) 2. doi:10.1186/1477-7827-10-2]. It is used clinically in the treatment of osteoporosis and heart disease [M.L. Stefanick, Estrogens and progestins: Background and history, trends in use, and guidelines and regimens approved by the US Food and Drug Administration, in: Am J Med, 2005. doi:10.1016/j.amjmed.2005.09.059]. Moreover, blockage of the E2 receptor with adjuvants such as tamoxifen for estrogen receptor positive tumours, in 10 which high estrogen helps the cancer cells grow and spread, is an effective method to reduce tumour vasculature used in clinics for many years especially for treatment of breast cancer [B. Fisher, J. Costantino, C. Redmond, R. Poisson, D. Bowman, J. Couture, N. V Dimitrov, N. Wolmark, D.L. Wickerham, E.R. Fisher, A randomized clinical trial evaluating tamoxifen in the treatment of patients with node-negative breast cancer who have estrogen-receptor-positive tumors., N Engl J Med. 320 (1989) 479-84. doi:10.1056/NEJM198902233200802, Early Breast Cancer Trialists Collaborative Group, Tamoxifen for early breast cancer: an overview of the randomised trials, Lancet. 351 (1998) 1451-1467. doi:10.1016/S0140-6736(97)11423-4]. E2 has been shown to promote endothelial cell migration and proliferation in vitro [K. Nikhil, S. Sharan, R. Wishard, S.R. Palla, R. Krishna Peddinti, P. Roy, Pterostilbene carboxaldehyde thiosemicarbazone, a resveratrol derivative inhibits 15 17β-Estradiol induced cell migration and proliferation in HUVECs, Steroids. 108 (2016) 17-30. doi:10.1016/j.steroids.2016.01.020, G.M. Rubanyi, A. Johns, K. Kauser, Effect of estrogen on endothelial function and angiogenesis, Vascul Pharmacol. 38 (2002) 89-98. doi:10.1016/S0306- 3623(02)00131-3] and to stimulate new blood vessel formation both in vitro and in vivo [D.E. Morales, K.A. McGowan, D.S. Grant, S. Maheshwari, D. Bhartiya, M.C. Cid, H.K. Kleinman, H. William Schnaper, Estrogen Promotes Angiogenic Activity in Human Umbilical Vein Endothelial Cells In Vitro and in a Murine Model, Circulation. 91 (1995) 755-63]. The inventors have confirmed that poly-L-lactic-acid (PLLA) scaffolds loaded with E2 were highly 15 angiogenic using the CAM assay [N. Mangir, C.J. Hillary, C.R. Chapple, S. MacNeil, Oestradiol-releasing Biodegradable 25 Mesh Stimulates Collagen Production and Angiogenesis: An Approach to Improving Biomaterial Integration in Pelvic Floor Repair, Eur Urol Focus. (2017). doi:10.1016/j.euf.2017.05.004].

E2 was previously reported as angiogenic both in vitro and in vivo by many groups as well 15 as our research group. Albrecht et al. suggested that E2 promotes angiogenesis through upregulation of VEGF. They reported rapidly increased VEGF expression and cell permeability by E2 administration to ovariectomized baboons [E.D. Albrecht, J.S. Babischkin, Y. Lidor, L.D. Anderson, L.C. Udoff, G.J. Pepe, Effect 15 of estrogen on angiogenesis in co-cultures of human endometrial cells and microvascular endothelial cells., Hum Reprod. 18 (2003) 2039-2047. doi:10.1093/humrep/deg415]. Similarly elevated VEGF mRNA expression levels were observed in ovariectomized rats after E2 treatment by Hyder et al. [S.M. Hyder, G.M. Stancel, C. Chiappetta, L. Murthy, H.L. Boettger-Tong, S. Makela, Uterine expression of vascular endothelial growth factor is increased by estradiol and 20 tamoxifen, Cancer Res. 56 (1996) 3954-3960]. In the same way, Morales et al. reported that E2 promoted migration of HUVECs and 20 formation of capillary-like networks on Matrigel [D.E. Morales, K.A. McGowan, D.S. Grant, S. Maheshwari, D. Bhartiya, M.C. Cid, H.K. Kleinman, H. William Schnaper, Estrogen Promotes Angiogenic Activity in Human Umbilical Vein Endothelial Cells In Vitro and in a Murine Model, Circulation. 91 (1995) 755-63]. Pence et at. indicated that exogenous E2 promoted endogenous production of VEGF by endometrial epithelial cells [J.C. Pence, K.B.H. Clancy, B.A.C. Harley, The induction of pro-angiogenic processes within a collagen scaffold via exogenous estradiol and endometrial epithelial cells, Biotechnol Bioeng. 112 (2015) 2185-2194. doi:10.1002/bit.25622]. More recently our group has demonstrated release of E2 from both biodegradable (PLA [N. Mangir, C.J. Hillary, C.R. Chapple, S. MacNeil, Oestradiol-releasing Biodegradable 25 Mesh Stimulates Collagen Production and Angiogenesis: An Approach to Improving Biomaterial Integration in Pelvic Floor Repair, Eur Urol Focus. (2017). doi:10.1016/j.euf.2017.05.004]) fibres and from nondegradable (PU [S. Shafaat, N. Mangir, S.R. Regureos, C.R. Chapple, S. MacNeil, Demonstration of improved tissue integration and angiogenesis with an elastic, estradiol releasing polyurethane material designed for use in pelvic floor repair, Neurourol Urodyn. (n.d.) n/a-n/a. doi:10.1002/nau.23510]) fibres with both electrospun scaffolds showing good pro angiogenic activity in the CAM assay.

As used herein the term "promoting wound healing" is to be understood as restoring a break in the continuity of skin tissue and includes disorders characterized by any disease, disorder, syndrome, anomaly, pathology, or abnormal condition of the skin (dermis and epidermis) and/or underlying connective tissue. For instance, wounds may comprise minor cuts or abrasions; partial thickness wounds; complicated/full thickness wounds; traumatic wounds such as e.g. abrasions, lacerations; surgical wounds; post-surgical incisions; chronic/non-healing wounds like pressure sores or non-healing diabetic foot wound; ulcers, in particular a venous ulcer, a decubitus, a skin ulcer derived from infection, a pressure ulcer or a diabetic ulcer; injuries to connective tissue like bone or cartilage; chemical or thermal burn injuries, in particular a third degree burn; an accidental wound; a necrotic wound like ischemic necrosis; an infected wound; a donor site of full thickness and split thickness skin graft; a bedsore; diabetes-induced and age-induced difficulty of skin surface wound healing and scars such as e.g. keloid scars, contracture scars, hypertrophic scars and acne scars. The wound may be an open wound or a closed wound. A 'closed' wound as used herein means a wound that was open at one point (e.g. a surgical incision or an accidental cut) and that has been purposefully closed by means of a suture, staple, surgical adhesive and the like.

As used herein the term "full thickness wound" refers to disruption of the dermis, tissue and disruption to the dermal blood vessels. In contrast "partial thickness wound" refers to disruption of the dermal tissue only.

One particular application of the D-deoxyribose or E2 (not claimed) is for use on extensive full thickness wounds due to burns injuries.

In accordance with the invention, wound healing can be achieved by administration to a subject any suitable dosage regimen, procedure and/or administration route of a composition comprising a D-deoxyribose sugar or E2 (not claimed) to a wound bed.

As used herein the term "wound bed" refers to the exposed surface on the body when a break in the continuity of the skin tissue has occurred.

Restoration of a wound can mean full healing or substantially full healing of the wound wherein the wounded tissue is restored to substantially the same state prior to wounding. Alternatively, restoration of a wound can mean partial healing of the wound. In a further alternative, restoration of a wound may include the reduction of formation of fibrous scar tissue. Wound healing may be for use in therapeutic or non-therapeutic [cosmetic] purposes.

As used herein, "induce" refers to the action of generating, promoting, forming, regulating or activating a particular phenomenon. As used herein, "increase" refers to the action of accelerating or enhancing a particular phenomenon.

D-deoxyribose or E2 (not claimed) may also be used to increase or induce angiogenesis in a wound bed.

Alternatively, D-deoxyribose or E2 (not claimed) may be used to increase or induce vascularisation in a wound bed.

As used herein "vascularisation" includes *de novo* formation, growth, development or proliferation of blood vessels derived from undifferentiated or under-differentiated cells.

Suitably, blood vessels may form by the *de novo* production of endothelial cells. As used herein "angiogenesis" refers to the formation of new vasculature (e.g., blood vessels; e.g., veins, arteries, venules, arterioles, capillaries) from pre-existing vasculature. For example, angiogenesis can occur by sprouting of new vessels from an existing vessel (sprouting angiogenesis), and/or by branching of a vessel (intussusceptive angiogenesis). Without wishing to be bound to any particular theory D-deoxyribose may induce chemical stimulation of angiogenesis via induction and/or stimulation and/or production of VEGF. VEGF is a known chemical stimulator of angiogenesis.

Alternatively, D-deoxyribose or E2 (not claimed) may be used in the treatment of hair loss. Suitably, D-deoxyribose or E2 (not claimed) can be used to increase or induce hair re-growth. D-deoxyribose or E2 (not claimed) may also be used to slow, prevent or minimise hair loss. It is contemplated that for this purpose D-deoxyribose may have both therapeutic and non-therapeutic [cosmetic] uses.

Suitably, D-deoxyribose or E2 (not claimed) may be used to treat alopecia. As used herein "alopecia" is a generic term which encompasses all forms of medical hair loss. Alopecia may be due to follicle absence, destruction (which may be scarring), disease, infection, shrinkage, disregulation (hair cycle alterations), or complete shut-down/dormancy. These changes can be temporary or permanent, partial, regional and/or apparently total.

Examples of specific types of alopecia that are encompassed by the use of the term alopecia are selected from the group consisting of: androgenic alopecia; alopecia areata; alopecia totalis; alopecia univeralis; telogen effluvium; anagen effluvium; traumatic alopecia, mechanical traction alopecia' from hairstyling routines; chemical-induced alopecia; heatinduced alopecia; radiation-induced alopecia; chemotherapy-induced alopecia; scarring alopecia; auto-immune disease induced alopecia (e.g. from discoid lupus erythematosus or chronic cutaneous lupus erythematosus); disease-related alopecia (e.g. from hyperthyroidism or hypothyroidism, iron deficiency); medication-induced alopecia (e.g. alopecia induced by antibiotics and antifungal drugs; antidepressants, anticonvulsants; anticoagulants such as heparin and some LMWH; NSAIDs such as aspirin; anti-hypertensives, hormone replacement therapy) and syphilitic alopecia.

Examples of other conditions which would be considered under this term are: hypopituitarism, e.g. Simmonds' disease and Sheehan's syndrome; other endocrinopathies e.g. hypothyroidism, diabetes mellitus, adrenal dyperplasia; chronic disease, e.g. lupus erythematosus, neoplasma; monilethrix; pseudomilethrix; hereditary hypotrichosis simplex of the scalp; congenital triangular temporal alopecia; alopecia of the cranial sutures - Hallermann Streiff's syndrome; hereditary cicatrical alopecia, e.g. Marie Unna type generalised hypotrichosis; diffuse congenital scarring follicular keratosis; epidermal nevus - benign epithelial hamartoma and/or malignant tumour or benign physical trauma, e.g. car accident, sporting injury; post-surgical; anti-tumoural agents, e.g. cytostatic and/or alkylating agents;chemical trauma, e.g. thioglycolates, anti-metabolics; ionizing radiation; toxic drugs, e.g. thallium, vitamin A; self-inflicted physical trauma, e.g. Trichotillomania; bacterial, e.g. Pyodermitis, Bockhart's impetigo; mycotic/fungal, e.g. candidiasis, dermatophylosia; accidental trauma/rubbing, e.g. hats, hairdressers etc. A related term is hypotrichosis which is defined as a decrease in the production of hair.

Suitably, a composition comprising a D-deoxyribose sugar or E2 (not claimed) may be applied to an isolated hair follicle. As used herein the term "hair follicle" refers to a tube-like structure originating from the epidermis where a hair fibre may develop. A hair follicle typically comprises the following structures: papilla, matrix, root sheath, sebaceous gland and optionally hair fibre (which comprises of a hair shaft and a hair root). The hair fibre may be of decreased diameter or not present at all, depending on the extent of alopecia or depending on the extent and stage of the formed follicle development.

Suitably, D-deoxyribose or E2 (not claimed) can be provided with a carrier. The carrier is preferably selected from a gel, more preferably a hydrogel, or a biocompatible matrix material, preferably an electrospun scaffold, a particle, a wound dressing or a swab that is impregnated with a solution containing D-deoxyribose or E2 (not claimed).

Carriers may consist of amorphous materials having no defined surfaces and lacking a specific shape or solid matrices.

Suitably, a carrier releasing D-deoxy ribose or E2 (not claimed) could act as a substitute for a dermis as it will stimulate new blood vessel ingrowth and provide a vascularised wound bed for subsequent grafting with a thin skin graft or cultured autologous keratinocytes to provide a permanent skin barrier layer.

The carrier may further comprise a substance that acts as an antimicrobial, for example silver or cerium.

These carriers may be suitable for sustained release of D-deoxyribose or E2 (not claimed) into the wound.

Preparations of D-deoxyribose or E2 (not claimed) with a carrier are especially suitable for topical administration.

Suitably, D-deoxyribose or E2 (not claimed) can be formulated with or coupled to a biocompatible matrix material. Matrix material as used herein means a carrier or a scaffold for cell recruitment, attachment, proliferation and differentiation and a potential delivery and storage device for D-deoxyribose or E2 (not claimed).

The preparation of biocompatible matrix materials are well known in the art. Biocompatible matrices preferably support wound healing and/or increase or induce angiogeneisis or vascularisation in a wound bed.

Suitably, biocompatible matrix materials may comprise a polymeric material. The polymeric material may comprise a homopolymer or a heteropolymer/copolymer of two or more different polymeric substances, or a combination of the two. As used herein, the term "polymer" refers to a macromolecule formed by the chemical bonding of five or more identical combining units called monomers e.g. sugars. Polymers may be naturally derived [e.g., polysaccharides, such as starch, cellulose, pectin, seaweed gums, vegetable gums; polypeptides, such as casein, albumin, globulin, keratin, insulin, DNA; and hydrocarbons], synthetic [such as thermoplastics (unvulcanized elastomers, nylon, polyvinyl chloride, poly (vinylidene fluoride trifluoroethylene) linear polyethylene, polystyrene, polypropylene, polyurethane, acrylate resins); thermosetting (e.g., vulcanized elastomers, crosslinked polyethylene, phenolics, alkyds, polyesters), and semisynthetic (e.g., cellulosics, such as rayon, methylcellulose, cellulose acetate; and modified starches)]. The term "homopolymer" refers to a natural or synthetic polymer derived from a single monomer. The term "heteropolymer" refers to a natural or synthetic polymer derived from more than one monomer subunit (i.e., co-polymer). Unless otherwise indicated, the term "polymer" is used generally to refer to both homopolymers and heteropolymers (i.e., copolymer) as described herein.

As used herein, the term "biocompatible material" or "biocompatible matrix material" means that the material does not stimulate a response or stimulates only a mild and/or transient response, as opposed to a severe or escalating response when the matrix material is placed or implanted at the desired wound bed.

Biocompatible matrix materials may include for example, synthetic or naturally occurring matrices such as collagen, acellular matrix, crosslinked biological scaffold molecules, tissue based bioengineered structural framework, biomanufactured bioprostheses, and other implanted structures such as for example, vascular grafts suitable for cell infiltration and proliferation useful in the promotion of wound healing. Additional suitable biocompatible matrix materials may include chemically modified collagenous tissue to reduce antigenicity and immunogenicity. Other suitable examples include collagen sheets for wound dressings, antigen-free or antigen reduced acellular matrix (Wilson G J et al. (1990) Trans Am Soc Artif Intern 36:340-343) or other biocompatible matrices which have been engineered to reduce the antigenic response to the xenograft material. Other matrices useful in promotion of wound healing may include for example, processed bovine pericardium proteins comprising insoluble collagen and elastin (Courtman DW et al. (1994) J Biomed Mater Res 28:655-666) and other acellular tissue which may be useful for providing a natural microenvironment for host cell migration to accelerate tissue regeneration (Malone J M et al. (1984) J Vasc Surg 1:181-91).

Suitably, the biocompatible matrix material can be formed by electrospinning to form a scaffold. The process of electrospinning involves application of a high voltage to an ejectable polymer solution to synthesise polymeric matrix materials. The high voltage applied is sufficient to overcome the surface tension of a polymeric solution such that it causes the polymer droplets to elongate thereby becoming fine fibres that form a non-woven matrix, mat or mesh with a random orientation. Electrospinning typically yields non-woven (i.e., mesh) matrices (also referred to as mats and/or scaffolds) with fibre diameters in the hundreds of nanometres range with interrelated pores. The electrospinning process typically leads to the formation of a smooth surface that is substantially free of any beaded fibres. The matrix fibres may comprise a non-woven mesh of random and/or aligned nanofibers or a combination thereof. Suitably, once loaded with the D-deoxyribose sugar the nanofibres will typically increase in diameter. For example, the loaded fibres may have a diameter of from about 0.2 microns to about 5.0 microns.

The electrospun nanofibers may comprise polycaprolactone. Advantageously polycaprolactone is biodegradable. Suitably, other biodegradable polymer(s) may be used instead of polycaprolactone. Examples of other suitable biodegradable polymers that could be suitable alternatives include polylactic acid (PLA), polyglycolide (PGA), poly(lactic-coglycolic acid) (PLGA) and poly(3-hydroxybutyrate-co-3-hydroxyvalerate) PHBV.

As used herein, the term a "biodegradable" material is one which is able to decompose under normal *in vivo* physiological conditions over a finite period into components which can be metabolized or excreted and have no harmful effects when decomposing *in vivo.* Alternatively, the carrier may be a hydrogel. Hydrogels comprise a network of hydrophilic functional groups attached to natural or synthetic polymeric backbone chains. They may comprise a colloidal gel with an aqueous dispersion medium. The protrusion of the hydrophilic functional groups from the polymeric chains makes hydrogels highly absorbent (some can contain over 99.9% water). The high liquid content of hydrogels allows the hydrogels to have a degree of flexibility comparable to natural tissue. The absorbent nature of hydrogels also allows for hydrogels to act as a reservoir for topical drug delivery. The hydrogel may be a cross-linked hydrogel. Cross-linking of the hydrogel can prevent dissolution. Cross-linking may be achieved by reacting with suitable cross-linking agents. Suitable hydrogels may include for example, three-dimensional networks of cross-linked hydrophilic polymers that are insoluble in water and interact with aqueous solutions by swelling. Exemplary hydrogels are highly conformable and permeable and can absorb varying amounts of aqueous solution, depending on their composition. Suitably, the hydrogel may be non-adhesive against the treatment site or treated for easy removal.

Suitably, a hydrogel may preferably comprise at least one of chitosan, gelatin, alginate, agarose, methylcellulose, collagen, hyaluronan or any combination thereof. Preferably, the hydrogel may comprise chitosan and collagen. Alternatively organo-chemical hydrogels may comprise polyvinyl alcohol, sodium polyacrylate, acrylate polymers and copolymers with an abundance of hydrophilic groups.

Suitably, the carrier comprises ascorbic acid or a derivative thereof such as, for example, L-ascorbic acid or acscorbate-2-phosphate (Mangir et al Acta Biomater. 2016 Jan 1; 29: 188-197.doi: 10.1016/i.actbio.2015.10.019)

The term "wound dressing" refers to a dressing for topical application to a wound and excludes compositions suitable for systemic administration. For example, the D-deoxyribose sugar or E2 (not claimed) may be dispersed in or on a solid sheet of wound contacting material such as a woven or nonwoven textile material, or may be dispersed in a layer of foam such as polyurethane foam, or in a hydrogel such as a polychitosan hydrogel, a polyvinyl alcohol, a polyacrylate hydrogel, gelatin, methyl cellulose, agarose, alginate, hyaluronic acid hydrogel or any combination thereof for example in a gel or ointment. Suitably the D-deoxyribose sugar or

E2 (not claimed) is dispersed in or on a biodegradable sheet material that provides sustained release of the active ingredients into the wound, for example a sheet of freeze-dried chitosan/polyvinyl alcohol mixture.

Suitably, D-deoxyribose or E2 (not claimed) may be provided in the form of a liquid, semi solid or solid composition for application directly, or the composition is applied to the surface of, or incorporated into, a solid contacting layer such as a wound dressing, hydrogel, scaffold or matrix. The dressing composition may be provided for example, in the form of a fluid or a gel. Suitable amorphous hydrogel dressings may include, for example, formulations of water, polymers and other ingredients with no shape, designed to donate moisture and to maintain a moist healing environments and or to rehydrate the wound site. The hydrogels may be used in combination with a secondary dressing cover.

Hydrogel dressings may include, for example, gauzes and non-woven sponges, ropes and strips saturated with an amorphous hydrogel. Suitable impregnated dressings may include, for example, gauzes and non-woven sponges, ropes and strips saturated with a solution, an emulsion, oil, gel or some other pharmaceutically active compound or carrier agent, including for example, saline, oil, zinc salts, petrolatum, xeroform and scarlet red as well as the compounds described herein.

Suitable silicone gel sheet dressings may include, for example, soft covers composed of crosslinked polymers reinforced with or bonded to mesh or fabric.

Suitable liquid dressings may include, for example, mixtures of multiprotein material and other elements found in the extracellular matrix. The solutions may be applied to the treatment site after debridement and cleansing and then covered with an absorbent dressing or a non-adherent pad.

Suitable transparent film dressings may include polymer membranes of varying thickness coated on one side with an adhesive. The transparent films may be impermeable to liquid, water and bacteria but permeable to moisture vapor and atmospheric gases. The transparency of the film may allow visualization of the treatment site.

Suitable filler dressings may include, for example, beads, creams, foams, gels, ointments, pads, pastes, pillows, powders, strands or other formulations. The fillers may be non-adherent and may include a time-released antimicrobial. Exemplary fillers may be useful to maintain a moist environment, manage exudate, and for treatment of for example, partial- and full-thickness wounds, infected wounds, draining wounds and deep wounds that require packing.

The D-deoxyribose or E2 (not claimed) may be provided in combination with conventional pharmaceutical excipients for topical application. Suitable pharmaceutical carriers include: pluronic gels, polaxamer gels, hydrogels containing cellulose derivatives, including hydroxyethyl cellulose, hydroxymethyl cellulose, carboxymethyl cellulose, hydroxypropylmethyl cellulose and mixtures thereof, and hydrogels containing polyacrylic acid (Carbopols).

Other suitable carriers also include creams/ointments used for topical pharmaceutical preparations, e.g., creams based on cetomacrogol emulsifying ointment. The above carriers may include alginate (as a thickener or stimulant), preservatives such as benzyl alcohol, buffers to control pH such as disodium hydrogen phosphate/sodium dihydrogen phosphate, agents to adjust osmolarity such as sodium chloride, and stabilizers such as EDTA.

Preferred formulations are formulations for topical administration. Also encompassed are compositions comprising incorporation of D-deoxyribose or E2 (not claimed) into liposomes, microemulsions, micelles, microparticles or vesicles for controlled delivery over an extended period of time to a wound bed.

The pharmaceutical composition may comprise D-deoxyribose or E2 (not claimed) formulated with or coupled to a biocompatible matrix, preferably comprising collagen, gelatin, chitosan and/or hyaluronan, as described above. The pharmaceutical composition may also comprise a hydrogel with D-deoxyribose or E2 (not claimed) as described above.

The pharmaceutical composition may comprise an antimicrobial agent, such as silver or cerium.

In particular, the present invention provides the pharmaceutical composition for use as a medicament. Particular uses are in wound healing and/or the stimulation of angiogenesis as described above. Alternative uses are for treatment of alopecia and other associated disorders as described above. A further alternative is for the cosmetic treatment of hair loss.

Also provided is the use of this method in vivo, in particular on a subject with a wound or in need of such a treatment, e.g. for stimulating angiogenesis.

"Topical administration" as used herein means administration to a definite surface, e.g. directly to the wound surface, or if the wound has been closed, to the area around or within the closure. As used herein, in accordance with all aspects of the invention, the term "subject" refers to vertebrates. Subject preferably refers to a mammalian animal, including a human, a veterinary or farm animal, a domestic animal or pet, and animals normally used for clinical research, including non-human primates, dogs, rats and mice. More specifically, the subject of the present invention may be a human.

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

### Example

### Experimental section 1Materials

Polycaprolactone (Mw: 45,000), L-rhamnose and NaOH were purchased from Sigma-Aldrich (USA). Chitosan (CS) was purchased from Mian Scientific Company, Lahore, Pakistan and further purified in our laboratories (degree of deacetylation (DD) 84%; Mw: 87047.26 g/mol) (Farooq et al., Materials Science and Engineering: C. 2015). Collagen was purchased from Mian Scientific suppliers Lahore. Poly(vinyl alcohol) (PVA) (Mw: 72,000, degree of hydrolysis 98%), hydrochloric acid (HCl) and sulfuric acid (H₂SO₄) were purchased from Merck (Germany). Triethyl orthoformate (98%) was purchased from Alfa Aesar (Germany). Glacial acetic acid (CH₃COOH) was purchased from AnalaR BDH Laboratory Supplies (UK). Ethanol (99.8%) was purchased from Sigma-Aldrich (Germany.) 2-deoxy-D-ribose, D-fucose and L-fucose were purchased from Sigma-Aldrich China, UK and Slovakia, respectively. 2-deoxy-L-ribose and D-rhamnose were products of Carbosynth (UK). Brain Heart Infusion broth and Tryptone soy broth (1% w/v) were purchased from Oxoid Ltd. Phenol red was purchased from Sigma-Aldrich UK.

### Electrospinning

PCL (10 wt%) was dissolved in 5 ml DMF/DCM (4:1, 10% w/v) and 2-deoxy-D-ribose (1.8 wt%) was dissolved into DMF (0.5 mL). Both solutions were combined and left on a rocking station overnight to produce a homogenous solution at room temperature. The following day, this solution was electrospun using 4×5mL syringes. These were placed horizontally on a programmable syringe pump (Kent Scientific, USA) and discharged at a rate of 40µL/min. The syringes were supplied with 17 kV using a high voltage power supply (Genvolt, UK). The fibrous scaffold was collected on a rotating drum 16 cmx6 cm in diameter at a distance of 17 cm from the needle tips. This drum rotated at 300 rpm, resulting in an 18 × 16 cm mat. Scaffolds were produced at a room temperature of around 20°C.

### Preparation of covalently crosslinked hydrogels: Chitosan/PVA Hydrogels Using Variable Amounts of Cross-Linking Agent for D-deoxysugar encapsulation

The CS (2.5% w/v) was dissolved in acetic acid (1%) solution and stirred magnetically for 12 hours. In a separate flask, PVA (10% w/v) was dissolved in distilled water at 80°C under magnetic stirring. Then the two solutions were mixed in 80:20 ratios of CS and PVA, respectively, and further stirred for 24 hours. The solution was then cast into separate petri dishes and the dishes were frozen at -80°C for 24 hours. The samples were lyophilized for 24 hours in a freeze dryer at -40°C. Then lyophilized hydrogels were rehydrated and soaked in different concentrations of TEOF (i.e. 0, 4, 8 and 16% w/v) with sulphuric acid (17% w/v) for 24 hours. The hydrogels were then removed from petri dishes and treated with NaOH (12% w/v) for one hour before washing with distilled water three times and lyophilizing for 24 hours.

### Preparation of cross-linked CS/collagen scaffolds by freeze gelation

CS and collagen (0.5g each) were dissolved in acetic acid (0.5M, 20mL) and stirred at room temperature until completely dissolved and a clear solution was obtained. To this, triethyl orthoformate (4% v/v, 0.8mL) was added as cross-linker and the solution was further stirred overnight for better homogeneity and cross-linking of materials. The mixture was then poured into a petri dish and frozen at -20°C overnight. The frozen membranes were then immersed in a pre-cooled ethanolic solution of sodium hydroxide (3M) and again placed at -20°C for 24 hours. After that, the frozen membranes were washed in 50% (v/v) ethanol solution and then with distilled water to remove the sodium hydroxide. The membranes were finally washed twice in absolute ethanol for 15 minutes and dried at room temperature to obtain porous scaffolds.

### FTIR analysis

The infrared (FT-IR) spectra were recorded on photo acoustic mode at the frequency range of 4000-400 cm⁻¹ with 256 consecutive scans at 8 cm⁻¹ resolution on a Thermo-Nicolet 6700 P FTIR Spectrometer (USA).

### Scanning electron microscopy (SEM)

The surface morphology of the composite matrices was examined under SEM, Model JEOL JSM 6480. The samples were gold sputtered coated prior to examination at a range of magnifications.

### Deoxysugar loading in scaffolds

For sugar loading, hydrogels were soaked in an aqueous solution of 2-deoxy-D-ribose (1 mg/ml) at 37°C until all the liquid had been absorbed by the hydrogels. Following storage at - 20°C overnight, hydrogels were dried in a lyophilizer (Christ, Alpha 1-2 LD plus, Germany) at -40°C before further characterization.

### Assessment of angiogenic properties of biomaterials using the CAM assay

Fertilized chicken (*Gallus domesticus*) eggs were purchased from Big Bird (Raiwind road, Lahore, Pakistan) and incubated at 37°C from day 2 of fertilization until day 8 in a humidified egg incubator (HHD, 435, China). At day 8, a square window (1 cm²) was cut into the shell and removed, and a 2 cm² piece of the electrospun scaffold or hydrogels (16% PCL electrospun scaffold/ CS/PVA hydrogel/ CS/collagen hydrogels) placed onto the CAM. Each egg was implanted with one scaffold or hydrogel only.

The shell window was closed with parafilm (Bemis Flexible Packaging, USA) and sealed with adhesive tape. After implantation, eggs were placed again at 37°C in a 40% humidified incubator until day 14. At day 14, biomaterials were retrieved and the eggs were sacrificed. Angiogenesis was quantified by taking light microscope pictures just before scaffold retrieval and then blindly scored by four assessors using histological images of the retrieved materials. The number of implanted and survived eggs is given in the caption of respective CAM assay figures.

### Full-thickness excisional rat wound model

For in vivo experiments, 20 mm diameter and 1.2 mm thickness circles of hydrogels were prepared. These samples were sterilized using ethanol (70%) solution, air dried and equilibrated in PBS briefly before placing on the wound. For animal trials, male Sprague-Dawley (SD) rats weighing 140-170 g were used in the study. The animals had free access to food and water and were kept in the animal house facility of the Centre of Excellence in Molecular Biology (CEMB), Lahore. All animals were treated according to procedures approved by the Institutional Animal Ethics Committee at CEMB, Lahore, Pakistan. Animals were anesthetized with ketamine (100 mg/kg body weight) and xylazine (10 mg/kg body weight) and then the fur from the dorsal side was removed with a hair trimmer (Dingling professional hair clipper, RF-608, China). The backs of the rats were completely shaved for adhesive tape fixation and dressing. After shaving, the backs were cleaned with ethanol and then they were transferred onto a clean sterilized table and three circular shaped wounds were created by removing skin at three marked areas with the help of surgical scissors (Noorani Surgical Medical Supplies, Pakistan). Immediately followed the excision process, one of these wounds was covered with sterilized, 2-deoxy-D-ribose -loaded (using 70% ethanol) membrane circles and the otherwas covered with the same material membranes but without any 2-deoxy-D-ribose. Both the implanted membranes were sutured in position using sterile braided silk sutures (Mersilk, diameter 220 microns). The scaffolds were covered with adhesive plaster (Mepore, Sweden), further dressed with cotton gauze and finally secured with white surgical adhesive tape (Nitto, Japan) to prevent damage from self-grooming. One of these wounds was kept open and covered with dressing only (Mepore, Sweden) to serve as a negative control.

Surgical dressings were removed after three days of membrane implantation. On days 0, 3, 9, 11, 14 and 17 post-wounding, the wounds were measured and photographed. The wound areas were quantified using Image j software. By the end of experiments, on day 17, rats were euthanized with an overdose of anaesthesia and tissue sections from implantation sites of normal, sham, control and 2-deoxy-D-ribose -loaded scaffold treated groups were removed. The samples were fixed in 3.7% paraformaldehyde (Sigma-Aldrich, USA) solution for 24 hours at room temperature. They were dehydrated using various concentrations of ethanol and water from 70% to 100% and paraffin blocks were made.

### Histology

Sections of 6 µm thick were cut from the paraffin embedded samples with a microtome (Leica TP 1020 Automatic Tissue Processor) and placed on Superfrost^{®} plus slides (Menzel-Gläser, Denmark). Conventional Haematoxylin and Eosin (H&E) staining and Gouldner's trichrome staining was performed (Yar et al., International Journal of Polymeric Materials and Polymeric Biomaterials. 2016) before mounting in DPX mounting medium (Fisher Scientific) with a coverslip.

For immunohistochemistry, 6 µm sections were processed with a mouse/rabbit specific HRP/DAB (ABC) Detection IHC Kit (Abcam). After rehydration, antigen retrieval and protein blocking steps, the sections were incubated with 3 different monoclonal antibodies (rabbit anti-CD34 (Abcam) 1:1000, mouse anti-CD80 (Santa Cruz) 1:100 and mouse anti-CD163 (AbD Serotec) 1:300) for 2 hours diluted in 1% BSA (Sigma-Aldrich). This was followed by 10 min incubation with a secondary biotinylated goat anti-polyvalent antibody (Abcam Detection IHC Kit). After incubation with an avidin and biotinylated horseradish peroxidase, the target proteins were visualized by incubation in peroxidase substrate and DAB chromogen (Abcam Detection IHC Kit). Samples were then counterstained with haematoxylin, dehydrated, and mounted as per H&E protocol. Controls consisted of samples incubated without primary and secondary antibodies, or incubated only with secondary antibodies. Semi-quantitative assessment of the extent of immunostaining was performed on a blinded observer basis using a qualitative grading scale; absent=0, mild presence=1, large presence=2, abundance=3, great abundance=4. Five representative images from each sample at each time point were assessed by two blinded researchers (n=10). Example photographs depicting 0, 1, 2, 3 and 4 were provided for reference and the median value from these scores was used. The M1/M2 ratio was also calculated for each using the values from the blind scoring of the immunostaining.

### Fermentation of sugars by bacteria.

To assess the ability of bacteria to metabolise the L and D deoxysugars studied in this investigation, their ability to ferment the sugars producing acid was determined.

### Bacteria

Five bacterial strains were employed in the study; four strains of *Staphylococcus aureus* and one strain of *Pseudomonas aeruginosa.* The *S*. *aureus* strains were S235 -a recent clinical isolate that was used in development of a bacterial infected skin model reported from this laboratory previously (Shepherd J et al., Tissue Engineering Part C: Methods. 2009). NCTC 6571 (Oxford) -a reference strain commonly used as a reference strain for antibiotic sensitivity testing, strain Newman -originating from a clinical strain but which has a defect in surface fibronectin binding protein, and L-9879 - another clinical isolate but which is hydrophilic.

### Growth and fermentation of sugars

Bacteria were grown in Brain Heart Infusion broth for 16h at 37 °C. Aliquots (100 ml) of Tryptone Soy broth (1% w/v) supplemented with phenol red (0.02% w/v; Sigma-Aldrich UK) and appropriate sugars (1% final conc.) were added to sterile 96 well plates. 5⊐ of the overnight broth cultures of each bacterial strain was then added to wells and incubated for 16h at 37 °C. The ability of each strain to ferment each sugar was judged by the production of acid and change of the phenol red pH indicator.

### Statistics

Differences between groups for the immunostaining were tested with a non-parametric Kruskal-Wallis test and multiple comparisons between individual groups using a Dunn's test.

### Results

### Incorporation of D-deoxysugar into electrospun PLC membranes

2-deoxy-D-ribose was encapsulated into electrospun nanofibers and DMF in DCM was used as the solvent for electrospinning. Electrospinning produced a sheet of sugar-loaded soft fibrous material (**Figure 1**). The average diameter of the PCL fibres without sugar was 224 nm ± 82 nm, whereas the average diameter of the 2-deoxy-D-ribose loaded fibers was significantly increased to 323 nm ± 25 nm (average diameter of 40 random fibers) (*p* ≤ 0.05). However, both types of fibres were randomly oriented with interrelated pores and their surfaces were smooth and free of any beads. After sugar loading, the colour of the membrane was light blue.

### Incorporation of D-sugar into Chitosan/PVA Hydrogels

The method of crosslinking between CS and PVA by using triethyl orthoformate (TEOF) was previously reported by our group in 2015 (Yar et al., Materials Science and Engineering: C. 2015; Shahzadi et al., Journal of biomaterials applications. 2016). In this study, we loaded 2-deoxy-D-ribose by physical adsorption onto the CS/PVA hydrogels crosslinked with 16% TEOF. 2-deoxy-D-ribose-loaded non-crosslinked hydrogel was used as a control in this study. The SEM micrographs showed that both cross-linking and sugar-loading appeared to affect the pore size and morphology of these hydrogels (Figure 2). Prior to cross-linking the hydrogels had a layered structure rather than interconnected pores. The average pore size was 103 µm ± 52 µm. After cross-linking the pore size was significantly reduced (*p*≤0.05). Addition of sugar in the absence of cross-linking changed the layered structure to distorted pores. With the introduction of both cross-linking and sugars the average pore size was significantly reduced to 68 µm ± 31 µm (*p*≤0.05). These new interconnected pores had a more uniform morphology and the pores were well-distributed throughout the hydrogel matrix.

### Incorporation of D-deoxyribose into triethyl orthoformate crosslinked chitosan/collagen hydrogels

Chitosan and collagen were crosslinked by using triethylorthoformate and then loaded with 2-deoxy-D-ribose by soaking in the aqueous sugar solution (1mg/ml). The average hydrogel thickness, before submersion in saline, was 1.11 mm and after 24 hours of submersion it increased to 1.37 mm. The values are the average of three independent experiments.

It is critical to assess the physical stabilty of the membranes as eventually the materials will be handed over to a surgeon for application to the skin. The surface of the membranes was smooth and devoid of any cracks. **Figure 3** below shows the physical appearance of this membrane along with all important parameters of strength, stretching and ability to be folded which will be advantageous during surgical procedures when applying to the wound site. Moreover, these membranes could be easily cut with a blade or scissors into the desired shape.

The membranes were strong and exhibited excellent flexibility, stretching and handling properties.

To investigate how different sugars affect the structure and morphology of hydrogels, which plays a critical role in cell infiltration and proliferation, the microscopic structures of these sugar-loaded hydrogels were examined by SEM (**Figure 4**). A sheet-like structure with an average pore size of 75.07 ± 34.48 µm was observed in the case of the control hydrogels **(****Figure 4a****).** The average pore-size of D and L-deoxyribose was 100.04 ± 24.01 µm and 93.33 ± 23.49 µm, respectively (**Figure 4b****, c**). In case of L-deoxyrhamnose (**Figure 4e**), the pore structure was intact, uniform and isotropic with average pore size of 110.89 ± 20.56 µm and its D isomer was not significantly different (average pore-size 102.45 ± 23.05 µm). D and L-fucose showed average cross-sectional pore-sizes of 72.18 ± 16.92 µm and 87.43 ± 14.11 µm respectively. Thus the SEM micrographs demonstrated that all the sugar-loaded hydrogels had similar highly porous structures.

### Characterization

### FTIR

FTIR spectroscopy was used to look at the interactions between the sugar and the microfibers of PCL. **Figure 5A** shows that the major peak for D-ribose seen at around 3400 cm⁻¹ was detected in the PCL fibers when this sugar was added to the polymer prior to spinning. A major absorbance band was observed at 1720 cm⁻¹ corresponding to the carbonyl ester of PCL. The peaks of alkyl groups, both in D-sugar and PCL, showed absorbance peaks between 2700-2900 cm⁻¹. The spectrum of D-sugar-loaded PCL fibers also shows that the sugar was distributed homogeneously in the electrospinning mixture.

The CS/PVA hydrogels, without (**Figure 5d**) and with (**Figure 5e**) cross-linker, contain the characteristic peaks of chitosan and PVA (**Figure 5B**). The peaks near 2900 cm⁻¹ were assigned to C-H stretching vibrations (Islam et al., Carbohydrate Polymers. 2012). A peak at 1530 cm⁻¹ was assigned to O-H bending vibrations of PVA (Li et al., Polymer. 2000). The bands which appeared near 1400 cm⁻¹ were due to C-H bending vibrations. The peaks at 1099 cm⁻¹ were due to C-O-C stretching vibrations. However, in the cross-linked hydrogel spectrum, O-H and N-H stretches also appeared at 3400-3200 cm⁻¹ (Li et al., Polymer. 2000; Teli et al., International journal of biological macromolecules. 2012) as a broad peak, but there was significant decrease in the intensity of this hump. This may be due to the utilization of -NH₂ of CS in forming new -C=N bond. The new peak of the imine bond appeared at 1630 cm⁻¹ (Yar et al., Materials Science and Engineering: C. 2015; Li et al., Polymer. 2000; Rokhade et al., Carbohydrate Polymers. 2007).

Figure **5C** shows the cross-linked CS/collagen spectra loaded with different sugars. The spectra show all the characteristic peaks of CS and collagen. A broad absorption band from 3200-3500 cm-1 was mainly due to O-H/N-H stretch vibrations. The broadness of the band was due to the intermolecular hydrogen bonding. Peaks between 2600-2800 cm⁻¹ appeared because of C-H stretching vibrations. Amide I peaks appeared at 1650 cm-1. The ether linkage C-O-C and C-O stretching vibrations were also shifted to 1147 cm⁻¹ and 1053 cm⁻¹. The sugar loading did not affect the FTIR peaks of hydrogels probably due to very low concentration of sugars in the polymer matrix.

### Assessment of pro-angiogenic responses to deoxy sugar-loaded biomaterials using the Chorionic Allantoic Membrane (CAM) assay.

### D-deoxyribose loaded electrospun fibers and hydrogels

The CAM assay was used to investigate the angiogenic potential of the synthesized materials. In the case of 2-deoxy-D-ribose-loaded fibers, we observed the growth of extensive new blood vessels under the material (see Figure **6A (b)**. This confirms the chemoattractant and angiogenic potential of the 2-deoxy-D-ribose-loaded materials.

2-deoxy-D-ribose was loaded into the chitosan/PVA triethyl orthoformate cross-linked hydrogels by soaking in an aqueous solution of D-deoxysugar. After drying in a lyophilizer the hydrogels were grafted into the fertilized eggs at day 8 and at day 14 the eggs were sacrificed. It was observed that the 2-deoxy-D-ribose-loaded scaffolds promoted angiogenesis as judged by observing more new blood vessels compared to the control material (Fig **6A****)**.

### D and L-sugar (3 sugars, 6 isomers) loaded chitosan/collagen cross-linked hydrogels

It is generally believed that biomaterials which help in the rapid growth and infiltration of blood vessels show better biocompatibility than those which exhibit delayed angiogenesis and continued inflammation (Wolf et al., Biomaterials. 2014). Consequently, this is an absolutely essential property for tissue engineering materials to ensure survival of the tissues.

D and L isomers of three -deoxysugars (ribose, fucose and rhamnose) were loaded onto the porous freeze gelated triethyl orthoformate (4%) covalently cross-linked chitosan/collagen (1:1) hydrogels. The hydrogels were soaked in an aqueous solution of sugars (1mg/ml) and were further lyophilized (freeze dried) to produce porous membranes. As for the electrospun scaffold, the hydrogels were grafted into the fertilized eggs at day 8 and after day 14 the eggs were sacrificed and the effect of sugars on angiogenesis was investigated.

As can be seen from Figure 7 all three of the L isomers stimulated angiogenesis but for the D isomers only the D isomer of deoxy - ribose was strongly angiogenic there was no significant response to the D isomer of deoxy-fucose and only a marginal response to the D isomer of deoxy-rhamnose.

### Assessment of wound healing responses to D-deoxyribose loaded hydrogel using a rat cutaneous wound model.

Wound healing in rats was accelerated by the application of 2-deoxy-D-ribose-loaded hydrogels. Full-thickness wounds were created by marking a 20mm area and then excisions were performed under anaesthetic using surgical scissors. The respective materials were applied to wounds, sutured in place and initially covered with Mepore (Sweden) adhesive bandages and finally with cotton bandages. All rats received the same dressings which were removed 3 days post-wounding. Each wound was photographed digitally at specific time points and further quantified using Image J software. Representative macroscopic pictures of wounds are shown in **Figure 8****.** Control hydrogels (cross-linked CS/collagen scaffold) and 2-deoxy-D-ribose-loaded hydrogels are shown at days 3, 9, 11, 14 and 17 post-wound.

At day 3 2-deoxy-D-ribose-loaded scaffolds were very much attached to the surrounding tissues as compared to the other tested materials (**Figure 8**) and when stretched manually showed good mechanical strength of the wounds. By day 9 the D-deoxyribose treated wounds were around 50% of the wound areas (**Figure 9**). At day 11 and 14 there was a further decrease in the wound area and for those treated with D-deoxyribose by day 17 the wound was completely closed in contrast to control wounds and those treated with hydrogels alone.

### Histological and immunohistochemistry analysis of excised wound tissues

Samples from all groups were harvested on day 17, and fixed overnight in paraformaldehyde. After dehydration samples were embedded with paraffin to make blocks which were sectioned at 6 µm. Slides were then stained with H&E, Gouldner's trichrome and with a DAB peroxidase kit looking at the detection of three antigens (CD34 for progenitor endothelial cells, CD80 for M1 macrophages and CD163 for M2 macrophages). Finally, the slides were covered with DPX mounting media and a glass coverslip prior to imaging with a light microscope. Sham (wound without any treatment), control (the wounds treated with chitosan and collagen scaffold) and D-dexoyribose (the wounds treated with scaffold loaded with 2-deoxy-D-ribose) groups were compared to normal skin from a non-treated rat.

At 17 days wound healing in the group treated with 2-deoxy-D-ribose was essentially complete and the structure of the explanted tissue was similar to normal skin as demonstrated by hematoxylin and eosin (H&E) staining (**Figure 10**). Goldren's Trichrome stains in blue/green collagen while muscle, epithelium, hair follicles and blood vessels are stained in red. Apart from the muscle layer stained under the skin as shown for the normal group, the rest looks very similar to the D-deoxyribose treated group with an epithelium and hair follicles already formed. In contrast, sham and control groups still showed disorganized granulation wound tissue by 17 days (**Figure 10**).

Macrophages expressing an M1 response markers are associated with implant rejection and activation of a chronic inflammatory response, while the M2 response markers are expressed by macrophages with an inflammatory response associated with constructive remodeling (Badylak et al., Tissue Engineering Part A. 2008).

As expected, there was no staining for M1 or M2 macrophages for the normal unwounded group (**Figure 11****)**. Some M1 and M2 macrophages were seen in the other groups as would be expected post wounding or post implantation of materials into animals. The table shown in **Figure 12** shows an assessment of the extent of macrophage presence undertaken blind by research staff in the laboratory. The ratio of M2 to M1 presence was around 1 for wounded animals and those treated with the unloaded chitosan. For those groups treated with chitosan loaded with 2-deoxy-D-ribose it was slightly (not statistically significant) greater (**Figure 11**).

### Ability of bacteria to metabolise L and D-deoxysugars.

The six sugars under investigation were compared to glucose with respect to acting as substrates for bacterial metabolism. Four strains of *S*. *aureus* were investigated and one strain of *Pseudomonas aeruginosa* as representatives of common pathogens in wound infections. Results with all four *S*. *aureus* strains were identical in that all could use glucose as a substrate for fermentation but none of these strains were able to use L- or D-isomers of the three deoxy sugars under investigation. *P*. *aeruginosa* was, however, able to ferment all sugars except for the L isomers of deoxy-fucose and deoxy-rhamnose.

The reason for investigating the ability of these sugars to act as substrates for bacteria is that if one is preparing a biomaterial to be used on burn wounds or chronic wounds then it would be preferable to use materials which could not be easily metabolised by bacteria.

**Table 1 The ability of bacteria to ferment sugars**

| **Bacteria** | **Deoxy -L-ribose** | **Deoxy-D-ribose** | **Deoxy -L-fucose** | **Deoxy-D-fucose** | **Deoxy-L-rhamnose** | **Deoxy-D-rhamnos e** | **Glucose** |
|---|---|---|---|---|---|---|---|
| *S*. *aureus* | | | | | | | |
| S235¹ | - | - | - | - | - | - | + |
| NCTC 6571² (Oxford) | - | - | - | - | - | - | + |
| Newman³ | - | - | - | - | - | - | + |
| L-9879⁴ | - | - | - | - | - | - | + |
| *P*. *aeruginosa* | + | + | - | + | - | + | + |

| | | | | | | | |
|---|---|---|---|---|---|---|---|
| ¹Clinical strain used previously in infection model ²A reference strain used for antibiotic sensitivity testing | | | | | | | |

### Conclusion

This study has demonstrated the pro-angiogenic ability of 2-deoxy-D-ribose when delivered either in electrospun PCL fibres or chitosan-based hydrogels. This was not shared by D-isomers of deoxy-fucose or deoxy-rhamnose although L-isomers of all three 2-deoxy-sugars tested were angiogenic. 2-deoxy-D-ribose could not be metabolised by four strains of *Staphlococcus aureus* although it could be metabolised by *Pseudomonas aeruginosa.* Addition of D-deoxy-ribose to a chitosan/collagen hydrogel accelerated wound healing and the restoration of hair follicles in full thickness wounds in rats.

### Experimental section 2

### Ex-ovo CAM assay

### Incubation of eggs

All CAM experiments were carried out according to the Home Office, UK guidelines. Schematic demonstration of the *ex-ovo* CAM assay is given in Figure 13. Fertilised chicken eggs (Gallus domesticus) were purchased from Henry Stewart & Co. (MedEggs, Norwich, UK). The eggs were carefully wiped with 20% industrial methylated spirit (IMS) solution using hand paper towels in order to remove dirt and feathers from the shell. The eggs were then incubated at 37.5°C until embryonic development day (EDD) 3, lying horizontally in an humidified egg incubator (RCOM King SURO, P&T Poultry, Powys, Wales).

### Transferring the embryos into petri dishes

On EDD3, the upper surface of the eggs were marked with a felt pen. The eggs were held horizontally (with the marked surface on top) and cracked on the edge of a 1000 ml glass beaker and kept close to the bottom surface of petri dishes. The embryos were then transferred gently into sterile petri dishes and kept in a humidified incubator (Binder, Tuttlingen, Germany) at 38°C.

### Application of substances onto CAM

Plastic rings (~6.5 mm in diameter) were used as a reservoir for substances and a marker for the implantation area, were placed on the CAM, and the substances were applied as 20µl volume onto the CAM twice a day to give a total volume of 40 µl between EDD7 and EDD11. Images of the rings were acquired using a digital microscope at EDD11, and then 20% lens culinaris agglutinin (LCA) (Vector Laboratories, Peterborough, UK) was injected into the circulation for labelling the intravascular endothelial cells using 30G needles under a dissection microscope (Wild Heerbrugg, Heerbrugg, Switzerland). CAMs were then removed and fixed in 3.7% formaldehyde solution. Embryos were sacrificed at the end of EDD11. Fixed CAM samples were then imaged under a confocal microscope (Zeiss LSM 510 Meta, Jena, Germany) for investigating the effect of substances on the microvascular structure of the CAMs.

### Quantification of angiogenesis

Blood vessels were quantified by segmentation of the images [C. Roma-Rodrigues, A. Heuer-Jungemann, A.R. Fernandes, A.G. Kanaras, P. V. Baptista, Peptide-coated gold nanoparticles for modulation of angiogenesis in vivo, IntJ Nanomedicine. 11 (2016) 2633-2639. doi:10.2147/IJN.S108661] so as to include all discernible vessels and then counting the branch points and calculating average vessel lengths [D. Ribatti, B. Nico, A. Vacca, M. Presta, The gelatin sponge-chorioallantoic membrane assay, Nat Protoc. 1 (2006) 85-91. doi:10.1038/nprot.2006.13, P. Brooks, A.P. Montgomery, D. Cheresh, Use of the 10-Day-Old Chick Embryo Model for Studying Angiogenesis, Integrin Protoc. 129 (1999) 257-269. doi:10.1385/1-59259-249-X:257] via multiple image processing steps. Firstly, the internal area of the ring was cropped, and the red, green and blue channels were split using Adobe Photoshop CS6 (ADOBE Systems Inc., San Jose, California, USA). The green channel was then imported to imaged (Wayne Rasband, National Institutes of Health, USA) for further analysis including unsharp mask filtering, enhancing the local contrast, noise removal and segmentation. Finally, the number of branch points was quantified using quantification software (AngioTool, National Cancer Institute, USA) (Figure 14A) and average blood vessel lengths were calculated by using binary image histograms with known pixel/mm ratios in imaged (Wayne Rasband, National Institutes of Health, USA).

The percentage vascular area (VA%) of the microvasculature of CAMs were quantified using confocal images of rhodamine labelled lectin injected CAM samples as shown in Figure 14B. To do this, images were imported to imaged (Wayne Rasband, National Institutes of Health, USA) and converted to binary images after filtering and smoothing processes prior to quantification. VA% was calculated using the histogram list of black and white areas in the image.

### Determination of the optimum concentration of E2 (not claimed) and 2dDR on CAM before loading scaffolds

All chemicals were purchased from Sigma Aldrich unless indicated otherwise. Recombinant VEGF165 stock solution was diluted to 2 ng/µl (VEGF-80ng) concentrations. E2 was dissolved in methanol then working solutions were prepared with phosphate buffered saline (PBS) so as to be (a) 100 ng/day (E2-100ng), (b) 200 ng/day (E2-200ng), and (b) 600 ng/day (E2-600ng) concentrations. 2dDR solutions were prepared by dissolving in PBS so as the final concentrations to be (a) 20 µg/day (2dDR-20), (b) 200 µg/day (2dDR-200), and (c) 1000 µg/day (2dDR-1000). Sunitinib malate (Sunitinib) was dissolved in DMSO and diluted with PBS with a final concentration of 50 ng/µl. Working solutions of all substances were prepared immediately before EDD7. Angiogenic efficiency of the different substance concentrations were evaluated by direct application of the substances to CAM (2 doses/day/embryo). Angiogenic activity was determined by quantifying the number of branch points and calculating the average blood vessel lengths.

### Comparison of the angiogenic potential of E2 (not claimed) and 2dDR releasing scaffolds on CAM

### Electrospinning E2 and 2dDR loaded PHBV scaffolds

### Preparation of the solutions

10% (w/w) PHBV solution loaded was prepared prior to electrospinning. 1 g of PHBV granules (Goodfellow, London, UK) were dissolved in 1 g of methanol (Fisher Scientific, Massachusetts, USA) and 8 g of DCM (Fisher Scientific, Massachusetts, USA) in a fume hood. Four 10% PHBV solutions were prepared prior to addition of substances. Finally, 25 mg E2, 50 mg of E2, 250 mg of 2dDR and 500 mg of 2dDR were then added to each solution per 1g of PHBV. The mixtures were magnetically stirred overnight.

### Electrospinning

Solutions (~10 ml) were loaded into 10 ml syringes fitted with 0.6 mm inner diameter syringe tips. Syringes were then placed in a syringe pump (GenieTMPlus, KentScientific, Connecticut, USA). Aluminium foil was used as the collector and placed at a distance of 17 cm from the needle tips. The pump was set to 40 µl/min, and 17 kV voltage was applied both to the collector and the tips. Electrospinning was done at room temperature until all the polymer solution was used.

### Scanning electron microscopy (SEM)

The surface morphology of E2 and 2dDR releasing scaffolds were observed under SEM (Philips/FEI XL-20 SEM; Cambridge, UK). The samples were coated with gold using a gold sputter (Edwards sputter coater S150B, Crawley, England) prior to imaging. Fiber diameter and pore sizes were measured using imaged.

### E2 and 2dDR release from the scaffolds

Scaffolds were cut into pieces so to fit into an 6-well plate, weighed and submerged in 4 ml of PBS. The E2 and 2dDR concentrations released from each group (25 mg E2, 50 mg E2, 250 mg 2dDR, 500 mg 2dDR) were measured fluorometrically using a UV-VIS spectrophotometer (Thermo Fischer Evolution 220, Massachusetts, USA) at 238 nm for 2dDR and 220 nm for E2. Absorbance values were converted into concentrations using a standard curve of known concentrations of E2 and 2dDR.

### Implantation of the E2 and 2dDR releasing scaffolds on CAM

Scaffolds were cut into 5.5 mm diameter circles using a laser cutting machine (Epilog Laser Cutter, Clevedon, UK) and sterilised under UV light for 1 hour prior to implantation. Two circular scaffolds were placed on CAM at EDD8. Images of the scaffolds were acquired using a digital microscope at EDD12 and EDD14. Microinjection of rhodamine labelled lectin into the circulation was performed, CAMs were removed and fixed in 3.7% formaldehyde solution. Embryos were then sacrificed at the end of EDD14. Angiogenesis was quantified by counting all blood vessels that converged towards the graft.

### Histological evaluation of the E2 and 2dDR releasing scaffolds on CAM

Haematoxylin and Eosin (H&E) staining was performed on cell impregnated scaffolds by modifiying a standard protocol [47]. Briefly, fixed samples were embedded in optimal cutting temperature OCT and frozen in liquid nitrogen for 3 minutes. Sections were cut 8-10 µm thick using a cryostat (Leica Biosystems Nussloch, Germany) at -20°C. Sections were then stained with haematoxylin for 90 seconds and eosin for 5 minutes respectively. Finally H&E images were acquired under a light microscope (Motic DM-B1, Xiamen, China). The total number of blood vessels adjacent to the scaffolds were quantified by counting blood vessels in H&E sections [A. Minajeva, M. Kase, M. Saretok, A. Adamson-Raieste, S. Kase, K. Niinepuu, M Vardja, T. Asser, J. Jaal, Impact of Blood Vessel Quantity and Vascular Expression of CD133 and ICAM-1 on Survival of Glioblastoma Patients, Neurosci J. 2017 (2017) 8 pages] Briefly, all discernible blood vessels adjacent to the scaffolds were counted by two independent researchers using two independent microscopes at 10× magnification from the total of six different slides for each group and six different area of interest from each slide.

### Comparison of the mechanical properties of E2 (not claimed) and 2dDR releasing scaffolds

Biomechanical testing samples were prepared by cutting 20 mm × 10 mm pieces from scaffolds. The clamps of the device were positioned 10 mm away from each other, and the width and thickness of each scaffold were measured. Test samples were clamped with two grips in a tensiometer (BOSE Electroforce Test Instruments, Minnesota, USA). Tensile tests were performed on each sample at a rate of 0.1 mm/s until the samples failed (n=4). The raw data of these tests were used for drawing stress-strain and load-displacement graphs. Ultimate tensile strength (UTS) was calculated from stress (σ) and stress (ε) curves of each sample, while stiffness was calculated from load (F) and displacement (ΔL) curve. Wettability tests of drug releasing electrospun scaffolds were also undertaken using a drop shape analyser (Krüss DSA100, Germany) under ambient laboratory conditions in order to see the effect of E2 and 2dDR on wettability of the scaffolds. In brief, a 5 µl droplet was dropped onto the scaffold surface, and the retention times of the droplet on scaffolds before complete absorption were calculated from recorded movies of the tests. The retention time for a minimum of three drops on three different substrates was measured for each type of sample.

### Statistics

Statistical analyses were performed using unpaired Student's t-test. P values <0.05 were considered as statistically significant, and the degree of significance was indicated with number of stars, **** P ≤ 0.0001, *** P ≤ 0.001, ** P ≤ 0.01, * P ≤ 0.05, ^{ns} P≥0.05.

### RESULTS

### Assessment of angiogenic activity of E2 (not claimed) and 2dDR on CAM

Quantification of the macroimages of CAMs showed that E2-100ng, E2-200ng and E2-600ng groups increased the number of branch points 1.3-fold, 1.5-fold and 1.4-fold respectively and, all concentrations increased the average vessel length 1.2-fold compared to controls over 4 days. In the same way, 2dDR-20µg and 2dDR-200µg increased the number of branch points by 1.3 times and 1.4 times, respectively. Both concentrations increased the average vessel length 1.2 times while there was no significant difference for the 2dDR-1000µg group compared to control scaffolds. Quantification of the branch points and average vessel lengths is given in Figure 15A and 15B respectively. Macroimages of the CAMs with the most effective concentrations of E2 and 2dDR are shown in Figure 16.

Microvascular evalutation of the CAM samples showed that VA% was increased from 55.3%± 3% to 79.5%± 5% and 71.7%± 3% for E2 and 2dDR applied groups respectively compared to controls over 4 days (Figure 16). VEGF and Sunitinib were used as positive and negative controls (Figure 16).

### Effect of including E2 (not claimed) and 2dDR on the microstructure of the PHBV scaffolds

SEM images of the E2 and 2dDR releasing PHBV scaffolds can be seen in Figure 17. The diameters of the fibres were significantly increased by addition of substances in all groups (25 mg E2 (0.83 ± 0.17 µm), 50 mg E2 (0.98 ± 0.35 µm), 250 mg 2dDR (0.89 ± 0.19 µm), 500 mg 2dDR (1.22 ± 0.28 µm)) added PHBV scaffolds when compared with the PHBV control group (0.66 ± 0.16 µm) as shown in the graph on the bottom right corner of Figure 17.

### Release of E2 (not claimed) and 2dDR from PHBV scaffolds over 30 days

The rate of release of E2 and 2dDR from scaffolds was assessed over 30 days as shown in Figure 18. By 7 days, 2dDR release from the scaffolds was 81.3% and 86.5% of the 2dDR present in the polymer solution for 250 mg and 500 mg 2dDR scaffolds, respectively (Figure 18A). In contrast the total E2 release from scaffolds within 7 days represented 1.3% and 1.6% of the initial E2 present in the polymer solution for 25 mg and 50 mg E2 scaffolds respectively (Figure 18B).

### Comparison of effects of E2 (not claimed) and 2dDR on the mechanical properties of scaffolds

As can be seen from Figure 19A, addition of all substances significantly increased the UTS of PHBV scaffolds when compared with plain PHBV scaffolds. The most significant increase in UTS was observed for 2dDR 250 mg group. Similarly the stiffness of the scaffolds loaded with 2dDR and E2 was significantly higher compared to unloaded PHBV scaffolds. 250 mg 2dDR loading increased the stiffness of PHBV scaffolds to the greatest extent as shown in Figure 19B. The wettability of the drug releasing scaffolds were investigated through a droplet retention time which is calculated using a drop shape analyser. Retention time of the water droplet on drug released scaffolds were given in Figure 19C. This showed that the addition of 2dDR made the scaffolds more wettable while the addition of E2 made the scaffolds less wettable.

### Assessment of angiogenic activity of the E2 (not claimed) and 2dDR releasing scaffolds on CAM

Assessment of E2 and 2dDR releasing scaffolds on CAM demonstrated that all groups at least doubled the number of discernible blood vessels growing towards the scaffolds in comparison with plain PHBV scaffolds as can be seen in Figure 20. Mean vessel counts for 25 mg E2 loaded scaffolds and 50 mg E2 loaded scaffolds were 49.5 (±0.92) (**** P ≤ 0.0001) and 37.9 (±1.05) (**** P ≤ 0.0001) respectively, while it was 48.6 (±1.02) (**** P ≤ 0.0001) and 37.1 (±1.37) (**** P ≤ 0.0001) for 250 mg and 500 mg 2dDR loaded scaffolds respectively when compared with control groups (mean vessel count: 23.1 (±1.24)). None of the loaded substances affected the embryo survival rate which was over 75% for each group.

### Histological Analysis of the E2 (not claimed) and 2dDR releasing scaffolds on CAM

The mean number of blood vessels adjacent to the scaffolds were significantly increased in response to all concentrations of both E2 and 2dDR releasing scaffolds when compared with controls and CAM only groups (see Figures 21 and 22).

All scaffolds whether loaded with pro-angiogenic agents or unloaded showed good attachment to the CAM, and all membranes showed similar cellular infiltration. Figure 21 shows representative histology images.

### CONCLUSION

From the data it can be concluded that both direct application of 2dDR and E2 and the gradual release of these factors from PHBV fibres stimulated angiogenesis in an *ex-ovo* CAM assay. These two small stable factors were readily incorporated into electrospun fibres such as those that would be used for tissue-engineered constructs and have great potential to be used for functionalisation of the TE scaffolds to promote angiogenesis *in vivo.*

### References

[1] Zhang Q, Hubenak J, lyyanki T, Alred E, Turza KC, Davis G, et al. Engineering vascularized soft tissue flaps in an animal model using human adipose-derived stem cells and VEGF+ PLGA/PEG microspheres on a collagen-chitosan scaffold with a flow-through vascular pedicle. Biomaterials. 2015;73:198-213.
[2] Miyagi Y, Chiu LL, Cimini M, Weisel RD, Radisic M, Li R-K. Biodegradable collagen patch with covalently immobilized VEGF for myocardial repair. Biomaterials. 2011;32:1280-90.
[3] Khojasteh A, Fahimipour F, Eslaminejad MB, Jafarian M, Jahangir S, Bastami F, et al. Development of PLGA-coated β-TCP scaffolds containing VEGF for bone tissue engineering. Materials Science and Engineering: C. 2016;69:780-8.
[4] Johnson KE, Wilgus TA. Vascular Endothelial Growth Factor and Angiogenesis in the Regulation of Cutaneous Wound Repair. Advances in Wound Care. 2014;3:647-61.
[5] Neufeld G, Cohen T, Gengrinovitch S, Poltorak Z. Vascular endothelial growth factor (VEGF) and its receptors. The FASEB journal. 1999;13:9-22.
[6] Long BL, Rekhi R, Abrego A, Jung J, Qutub AA. Cells as state machines: cell behavior patterns arise during capillary formation as a function of BDNF and VEGF. Journal of theoretical biology. 2013;326:43-57.
[7] Xin H, Zhong C, Nudleman E, Ferrara N. Evidence for Pro-angiogenic Functions of VEGF-Ax. Cell. 2016;167:275-84. e6.
[8] Chiu LL, Radisic M. Scaffolds with covalently immobilized VEGF and Angiopoietin-1 for vascularization of engineered tissues. Biomaterials. 2010;31:226-41.
[9] Çak 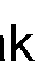 r-Özkan N, E 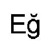 ri S, Bekar E, Altunkaynak BZ, Kabak YB, Kivrak EG. The Use of Sequential VEGF-and BMP2-Releasing Biodegradable Scaffolds in Rabbit Mandibular Defects. Journal of Oral and Maxillofacial Surgery. 2017;75:221. e1-. e14.
[10] Zhang Y, Huang J, Huang L, Liu Q, Shao H, Hu X, et al. Silk Fibroin-Based Scaffolds with Controlled Delivery Order of VEGF and BDNF for Cavernous Nerve Regeneration. ACS Biomaterials Science & Engineering. 2016;2:2018-25.
[11] Zhao L, Ma S, Pan Y, Zhang Q, Wang K, Song D, et al. Functional Modification of Fibrous PCL Scaffolds with Fusion Protein VEGF-HGFI Enhanced Cellularization and Vascularization. Advanced healthcare materials. 2016;5:2376-85.
[12] Wu J, Ye J, Zhu J, Xiao Z, He C, Shi H, et al. Heparin-based coacervate of FGF2 improves dermal regeneration by asserting a synergistic role with cell proliferation and endogenous facilitated VEGF for cutaneous wound healing. Biomacromolecules. 2016;17:2168-77.
[13] Gigliobianco G, Chong CK, MacNeil S. Simple surface coating of electrospun poly-L-lactic acid scaffolds to induce angiogenesis. Journal of biomaterials applications. 2015;30:50-60.
[14] Gilmore L, Rimmer S, McArthur SL, Mittar S, Sun D, MacNeil S. Arginine functionalization of hydrogels for heparin binding-a supramolecular approach to developing a pro-angiogenic biomaterial. Biotechnology and bioengineering. 2013;110:296-317.
[15] Easton C, Bullock A, Gigliobianco G, McArthur S, MacNeil S. Application of layer-by-layer coatings to tissue scaffolds-development of an angiogenic biomaterial. Journal of Materials Chemistry B. 2014;2:5558-68.
[16] Ferrara N, Gerber H-P, LeCouter J. The biology of VEGF and its receptors. Nature medicine. 2003;9:669-76.
[17] Harmey JH. VEGF and Cancer: Springer Science & Business Media; 2004.
[18] Hicklin DJ, Ellis LM. Role of the vascular endothelial growth factor pathway in tumor growth and angiogenesis. Journal of clinical oncology. 2005;23:1011-27.
[19] Tan Q, Chen B, Yan X, Lin Y, Xiao Z, Hou X, et al. Promotion of diabetic wound healing by collagen scaffold with collagen-binding vascular endothelial growth factor in a diabetic rat model. Journal of tissue engineering and regenerative medicine. 2014;8:195-201.
[20] Xie Z, Paras CB, Weng H, Punnakitikashem P, Su L-C, Vu K, et al. Dual growth factor releasing multi-functional nanofibers for wound healing. Acta biomaterialia. 2013;9:9351-9.
[21] Guo R, Xu S, Ma L, Huang A, Gao C. The healing of full-thickness burns treated by using plasmid DNA encoding VEGF-165 activated collagen-chitosan dermal equivalents. Biomaterials. 2011;32:1019-31.
[22] Simón-Yarza T, Formiga FR, Tamayo E, Pelacho B, Prosper F, Blanco-Prieto MJ. Vascular Endothelial Growth Factor-Delivery Systems for Cardiac Repair: An Overview. Theranostics. 2012;2:541-52.
[23] Thompson M. Oxford Textbook of Vascular Surgery: Oxford University Press; 2016.
[24] Shahzad SA, Yar M, Bajda M, Jadoon B, Khan ZA, Naqvi SAR, et al. Synthesis and biological evaluation of novel oxadiazole derivatives: A new class of thymidine phosphorylase inhibitors as potential anti-tumor agents. Bioorganic & Medicinal Chemistry. 2014;22:1008-15.
[25] Christensen M, Borza T, Dandanell G, Gilles A-M, Barzu O, Kelln RA, et al. Regulation of expression of the 2-deoxy-D-ribose utilization regulon, deoQKPX, from Salmonella enterica serovar typhimurium. Journal of bacteriology. 2003;185:6042-50.
[26] Farooq A, Yar M, Khan AS, Shahzadi L, Siddiqi SA, Mahmood N, et al. Synthesis of piroxicam loaded novel electrospun biodegradable nanocomposite scaffolds for periodontal regeneration. Materials Science and Engineering: C. 2015;56:104-13.
[27] Yar M, Gigliobianco G, Shahzadi L, Dew L, Siddiqi SA, Khan AF, et al. Production of chitosan PVA PCL hydrogels to bind heparin and induce angiogenesis. International Journal of Polymeric Materials and Polymeric Biomaterials. 2016;65:466-76.
[28] Shepherd J, Douglas I, Rimmer S, Swanson L, MacNeil S. Development of three-dimensional tissue-engineered models of bacterial infected human skin wounds. Tissue Engineering Part C: Methods. 2009;15:475-84.
[29] Yar M, Shahzad S, Siddiqi SA, Mahmood N, Rauf A, Anwar MS, et al. Triethyl orthoformate mediated a novel crosslinking method for the preparation of hydrogels for tissue engineering applications: characterization and in vitro cytocompatibility analysis. Materials Science and Engineering: C. 2015;56:154-64.
[30] Shahzadi L, Yar M, Jamal A, Siddiqi SA, Chaudhry AA, Zahid S, et al. Triethyl orthoformate covalently cross-linked chitosan-(poly vinyl) alcohol based biodegradable scaffolds with heparin-binding ability for promoting neovascularisation. Journal of biomaterials applications. 2016:0885328216650125.
[31] Islam A, Yasin T. Controlled delivery of drug from pH sensitive chitosan/poly (vinyl alcohol) blend. Carbohydrate Polymers. 2012;88:1055-60.
[32] Li X, Goh S, Lai Y, Wee A. Miscibility of carboxyl-containing polysiloxane/poly (vinylpyridine) blends. Polymer. 2000;41:6563-71.
[33] Teli M, Sheikh J. Extraction of chitosan from shrimp shells waste and application in antibacterial finishing of bamboo rayon. International journal of biological macromolecules. 2012;50:1195-200.
[34] Rokhade AP, Patil SA, Aminabhavi TM. Synthesis and characterization of semiinterpenetrating polymer network microspheres of acrylamide grafted dextran and chitosan for controlled release of acyclovir. Carbohydrate Polymers. 2007;67:605-13.
[35] Wolf MT, Dearth CL, Ranallo CA, LoPresti ST, Carey LE, Daly KA, et al. Macrophage polarization in response to ECM coated polypropylene mesh. Biomaterials. 2014;35:6838-49.
[36] Badylak SF, Valentin JE, Ravindra AK, McCabe GP, Stewart-Akers AM. Macrophage phenotype as a determinant of biologic scaffold remodeling. Tissue Engineering Part A. 2008; 14:1835-42.
[37] Chua AWC, Khoo YC, Tan BK, Tan KC, Foo CL, Chong SJ. Skin tissue engineering advances in severe burns: review and therapeutic applications. Burns & trauma. 2016;4:3.
[38] Blais M, Parenteau-Bareil R, Cadau S, Berthod F. Concise Review: Tissue-Engineered Skin and Nerve Regeneration in Burn Treatment. Stem cells translational medicine. 2013;2:545-51.
[39] Yi JW, Kim JK. Prospective randomized comparison of scar appearances between cograft of acellular dermal matrix with autologous split-thickness skin and autologous split-thickness skin graft alone for full-thickness skin defects of the extremities. Plastic and reconstructive surgery. 2015;135:609e-16e.
[40] Sharma K, Bullock A, Ralston D, MacNeil S. Development of a one-step approach for the reconstruction of full thickness skin defects using minced split thickness skin grafts and biodegradable synthetic scaffolds as a dermal substitute. Burns. 2014;40:957-65.
[41] Böttcher-Haberzeth S, Biedermann T, Reichmann E. Tissue engineering of skin. Burns. 2010;36:450-60.
[42] Boyce ST, Kagan RJ, Yakuboff KP, Meyer NA, Rieman MT, Greenhalgh DG, et al. Cultured skin substitutes reduce donor skin harvesting for closure of excised, full-thickness burns. Annals of surgery. 2002;235:269-79.
[43] Supp DM, Boyce ST. Engineered skin substitutes: practices and potentials. Clinics in dermatology. 2005;23:403-12.
[44] Laschke MW, Harder Y, Amon M, Martin I, Farhadi J, Ring A, et al. Angiogenesis in tissue engineering: breathing life into constructed tissue substitutes. Tissue engineering. 2006;12:2093-104.
[45] Hacker S, Mittermayr R, Nickl S, Haider T, Lebherz-Eichinger D, Beer L, et al. Paracrine factors from irradiated peripheral blood mononuclear cells improve skin regeneration and angiogenesis in a porcine burn model. Scientific reports. 2016;6.
[46] Nguyen DQ, Potokar TS, Price P. An objective long-term evaluation of Integra (a dermal skin substitute) and split thickness skin grafts, in acute burns and reconstructive surgery. Burns. 2010;36:23-8.
[47] Weigert R, Choughri H, Casoli V. Management of severe hand wounds with Integra® dermal regeneration template. Journal of Hand Surgery (European Volume). 2011;36:185-93.
[48] Cleland H, Wasiak J, Dobson H, Paul M, Pratt G, Paul E, et al. Clinical application and viability of cryopreserved cadaveric skin allografts in severe burn: a retrospective analysis. Burns. 2014;40:61-6.
[49] MacNeil S. Progress and opportunities for tissue-engineered skin. Nature. 2007;445:874-80.
[50] Sahota PS, Burn JL, Heaton M, Freedlander E, Suvarna SK, Brown NJ, et al. Development of a reconstructed human skin model for angiogenesis. Wound repair and regeneration. 2003;11:275-84.

## Claims

1. A D-deoxyribose sugar for use in promoting wound healing wherein the sugar is provided in a carrier and wherein the carrier is a biocompatible matrix material or a hydrogel.

2. The D-deoxyribose sugar for use according to claim 1, wherein the D-deoxyribose is 2-deoxyribose.

3. The D-deoxyribose sugar for use according to claim 1 or 2, wherein the carrier is a biodegradable carrier.

4. The D-deoxyribose sugar for use according to any one of claims 1 to 3, wherein the matrix material is an electrospun scaffold, optionally wherein the electrospun scaffold comprises at least one of polylactic acid (PLA), polyglycolide (PGA), poly(lactic-co-glycolic acid) (PLGA) or poly(3-hydroxybutyrate-co-3-hydroxyvalerate) PHBV.

5. The D-deoxyribose sugar for use according to claim 1 or 2, wherein the hydrogel is a crosslinked hydrogel.

6. The D-deoxyribose sugar for use according to claim 1 or claim 5, wherein the hydrogel comprises at least one of chitosan, gelatin, alginate, agarose, methylcellulose, hyaluronan or any combination thereof, optionally:
(i) wherein the hydrogel comprises chitosan and collagen; and/or
(ii) wherein the hydrogel comprises polyvinyl alcohol, sodium polyacrylate, acrylate polymers or any combination thereof.

7. The D-deoxyribose sugar for use according to any one of claims 5 to 6, wherein the hydrogel comprises chitosan and polyvinyl alcohol.

8. The D-deoxyribose sugar for use according to any one of the preceding claims, wherein the carrier further comprises an antimicrobial agent.

9. The D-deoxyribose sugar for use according to any one of the preceding claims, wherein the wound is a chronic wound.

10. The D-deoxyribose sugar for use according to any one of the preceding claims, wherein the wound is a full thickness wound.

11. The D-deoxyribose sugar for use according to any one of the preceding claims, wherein the wound is a burn injury.

12. A carrier comprising a sugar, wherein the sugar consists of D-deoxyribose sugar, and wherein the carrier is a biocompatible matrix material or a hydrogel.

13. The carrier according to claim 12, wherein the biocompatible matrix material is an electrospun scaffold, optionally wherein the electrospun scaffold comprises at least one of polylactic acid (PLA), polyglycolide (PGA), poly(lactic-co-glycolic acid) (PLGA) or poly(3-hydroxybutyrate-co-3-hydroxyvalerate) PHBV.

14. The carrier I according to claim 12, wherein the hydrogel comprises at least one of chitosan, gelatin, alginate, agarose, methylcellulose, hyaluronan or any combination thereof.

15. The carrier according to claim 12 or 14 wherein the hydrogel comprises chitosan and collagen.

16. The carrier according to any one of claims 12, 14 or 15, wherein hydrogel comprises polyvinyl alcohol, sodium polyacrylate, acrylate polymers or any combination thereof.

17. The carrier according to any one of claims 12, or 14 to 16, wherein the hydrogel comprises chitosan and polyvinyl alcohol.

18. The carrier of any one of claims 12 or 13 for use in promoting wound healing or for use in a method of increasing or inducing vascularization in a wound bed, wherein the carrier is a biocompatible matrix material.

19. The carrier of any one of claims 12, or 14 to 17 for use in promoting wound healing or for use in a method of increasing or inducing angiogenesis in a wound bed, wherein the carrier is a hydrogel.

20. A wound dressing comprising the carrier according to any one of claims 12 to 17.

## Patentansprüche

1. Ein D-Desoxyribose-Zucker zur Verwendung bei der Förderung der Wundheilung, wobei der Zucker in einem Träger bereitgestellt wird und wobei der Träger ein biokompatibles Matrixmaterial oder ein Hydrogel ist.

2. Der D-Desoxyribose-Zucker zur Verwendung gemäß Anspruch 1, wobei die D-Desoxyribose 2-Desoxyribose ist.

3. Der D-Desoxyribose-Zucker zur Verwendung gemäß Anspruch 1 oder 2, wobei der Träger ein biologisch abbaubarer Träger ist.

4. Der D-Desoxyribose-Zucker zur Verwendung gemäß einem der Ansprüche 1 bis 3, wobei das Matrixmaterial ein elektrogesponnener Scaffold ist, optional wobei der elektrogesponnene Scaffold mindestens eines von Polymilchsäure (PLA), Polyglycolid (PGA), Poly(milch-coglycolsäure) (PLGA) oder Poly(3-hydroxybutyrat-co-3-hydroxyvalerat) PHBV umfasst.

5. Der D-Desoxyribose-Zucker zur Verwendung gemäß Anspruch 1 oder 2, wobei das Hydrogel ein vernetztes Hydrogel ist.

6. Der D-Desoxyribose-Zucker zur Verwendung gemäß Anspruch 1 oder Anspruch 5, wobei das Hydrogel mindestens eine von Chitosan, Gelatine, Alginat, Agarose, Methylcellulose, Hyaluronan oder eine beliebige Kombination davon umfasst, optional:
(i) wobei das Hydrogel Chitosan und Kollagen umfasst; und/oder
(ii) wobei das Hydrogel Polyvinylalkohol, Natriumpolyacrylat, Acrylatpolymere oder eine beliebige Kombination davon umfasst.

7. Der D-Desoxyribose-Zucker zur Verwendung gemäß einem der Ansprüche 5 bis 6, wobei das Hydrogel Chitosan und Polyvinylalkohol umfasst.

8. Der D-Desoxyribose-Zucker zur Verwendung gemäß einem der vorhergehenden Ansprüche, wobei der Träger weiterhin ein antimikrobielles Mittel umfasst.

9. Der D-Desoxyribose-Zucker zur Verwendung gemäß einem der vorhergehenden Ansprüche, wobei die Wunde eine chronische Wunde ist.

10. Der D-Desoxyribose-Zucker zur Verwendung gemäß einem der vorhergehenden Ansprüche, wobei die Wunde eine Vollhautwunde ist.

11. Der D-Desoxyribose-Zucker zur Verwendung gemäß einem der vorhergehenden Ansprüche, wobei die Wunde eine Verbrennungsverletzung ist.

12. Ein Träger umfassend einen Zucker, wobei der Zucker aus D-Desoxyribose-Zucker besteht, und wobei der Träger ein biokompatibles Matrixmaterial oder ein Hydrogel ist.

13. Der Träger gemäß Anspruch 12, wobei das biokompatible Matrixmaterial ein elektrogesponnener Scaffold ist, optional wobei der elektrogesponnene Scaffold mindestens eines von Polymilchsäure (PLA), Polyglycolid (PGA), Poly(milch-co-glycolsäure) (PLGA) oder Poly(3-hydroxybutyrat-co-3-hydroxyvalerat) PHBV umfasst.

14. Der Träger I gemäß Anspruch 12, wobei das Hydrogel mindestens eines von Chitosan, Gelatine, Alginat, Agarose, Methylcellulose, Hyaluronan oder eine Kombination davon umfasst.

15. Der Träger gemäß Anspruch 12 oder 14, wobei das Hydrogel Chitosan und Kollagen umfasst.

16. Der Träger gemäß einem der Ansprüche 12, 14 oder 15, wobei das Hydrogel Polyvinylalkohol, Natriumpolyacrylat, Acrylatpolymere oder eine Kombination davon umfasst.

17. Der Träger gemäß einem der Ansprüche 12 oder 14 bis 16, wobei das Hydrogel Chitosan und Polyvinylalkohol umfasst.

18. Der Träger gemäß einem der Ansprüche 12 oder 13 zur Verwendung bei der Förderung der Wundheilung oder zur Verwendung bei der Erhöhung oder Indizierung der Vaskularisierung in einem Wundbett, wobei der Träger ein biokompatibles Matrixmaterial ist.

19. Der Träger gemäß einem der Ansprüche 12 oder 14 bis 17 zur Verwendung bei der Förderung der Wundheilung oder zur Verwendung bei der Erhöhung oder Indizierung der Angiogenese in einem Wundbett, wobei der Träger ein Hydrogel ist.

20. Ein Wundverband umfassend den Träger gemäß einem der Ansprüche 12 bis 17.

## Revendications

1. - Sucre D-désoxyribose pour une utilisation pour favoriser la cicatrisation des plaies, dans lequel le sucre est fourni dans un support et dans lequel le support est un matériau de matrice biocompatible ou un hydrogel.

2. - Sucre D-désoxyribose pour une utilisation selon la revendication 1, dans lequel le D-désoxyribose est le 2-désoxyribose.

3. - Sucre D-désoxyribose pour une utilisation selon l'une des revendications 1 ou 2, dans lequel le support est un support biodégradable.

4. - Sucre D-désoxyribose pour une utilisation selon l'une quelconque des revendications 1 à 3, dans lequel le matériau de matrice est un échafaudage électrofilé, facultativement dans lequel l'échafaudage électrofilé comprend au moins l'un parmi l'acide polylactique (PLA), le polyglycolide (PGA), l'acide poly(lactique-co-glycolique) (PLGA) ou le poly(3-hydroxybutyrate-co-3-hydroxyvalérate) PHBV.

5. - Sucre D-désoxyribose pour une utilisation selon l'une des revendications 1 ou 2, dans lequel l'hydrogel est un hydrogel réticulé.

6. - Sucre D-désoxyribose pour une utilisation selon la revendication 1 ou la revendication 5, dans lequel l'hydrogel comprend au moins l'un parmi le chitosan, la gélatine, l'alginate, l'agarose, la méthylcellulose, l'hyaluronane ou toute combinaison de ceux-ci, facultativement :
(i) dans lequel l'hydrogel comprend du chitosan et du collagène ; et/ou
(ii) dans lequel l'hydrogel comprend de l'alcool polyvinylique, du polyacrylate de sodium, des polymères d'acrylate ou toute combinaison de ceux-ci.

7. - Sucre D-désoxyribose pour une utilisation selon l'une quelconque des revendications 5 et 6, dans lequel l'hydrogel comprend du chitosan et de l'alcool polyvinylique.

8. - Sucre D-désoxyribose pour une utilisation selon l'une quelconque des revendications précédentes, dans lequel le support comprend en outre un agent antimicrobien.

9. - Sucre D-désoxyribose pour une utilisation selon l'une quelconque des revendications précédentes, dans lequel la plaie est une plaie chronique.

10. - Sucre D-désoxyribose pour une utilisation selon l'une quelconque des revendications précédentes, dans lequel la plaie est une plaie de pleine épaisseur.

11. - Sucre D-désoxyribose pour une utilisation selon l'une quelconque des revendications précédentes, dans lequel la plaie est une brûlure.

12. - Support comprenant un sucre, dans lequel le sucre consiste en un sucre D-désoxyribose, et dans lequel le support est un matériau de matrice biocompatible ou un hydrogel.

13. - Support selon la revendication 12, dans lequel le matériau de matrice est un échafaudage électrofilé, facultativement dans lequel l'échafaudage électrofilé comprend au moins l'un parmi l'acide polylactique (PLA), le polyglycolide (PGA), l'acide poly(lactique-co-glycolique) (PLGA) ou le poly(3-hydroxybutyrate-co-3-hydroxyvalérate) PHBV.

14. - Support selon la revendication 12, dans lequel l'hydrogel comprend au moins l'un parmi le chitosan, la gélatine, l'alginate, l'agarose, la méthylcellulose, l'hyaluronane ou toute combinaison de ceux-ci.

15. - Support selon l'une des revendications 12 ou 14, dans lequel l'hydrogel comprend du chitosan et du collagène.

16. - Support selon l'une quelconque des revendications 12, 14 ou 15, dans lequel l'hydrogel comprend de l'alcool polyvinylique, du polyacrylate de sodium, des polymères d'acrylate ou toute combinaison de ceux-ci.

17. - Support selon l'une quelconque des revendications 12, ou 14 à 16, dans lequel l'hydrogel comprend du chitosan et de l'alcool polyvinylique.

18. - Support selon l'une quelconque des revendications 12 ou 13, pour une utilisation pour favoriser la cicatrisation des plaies ou pour une utilisation dans une méthode d'augmentation ou d'induction de vascularisation dans un lit de plaie, dans lequel le support est un matériau de matrice biocompatible.

19. - Support selon l'une quelconque des revendications 12, ou 14 à 17, pour une utilisation pour favoriser la cicatrisation des plaies ou pour une utilisation dans une méthode d'augmentation ou d'induction d'angiogenèse dans un lit de plaie, dans lequel le support est un hydrogel.

20. - Pansement comprenant le support selon l'une quelconque des revendications 12 à 17.
